(19) Europäisches Patentamt
European Patent Office
Office européen des brevets

(11) EP 0 590 919 B1

# (12) EUROPEAN PATENT SPECIFICATION

(45) Date of publication and mention of the grant of the patent:
**29.12.1999 Bulletin 1999/52**

(51) Int Cl.[6]: **C07D 473/06**, C07D 473/08, C07D 473/12, A61K 31/52

(21) Application number: **93307654.9**

(22) Date of filing: **28.09.1993**

## (54) Therapeutic agents for parkinson's disease

Therapeutische Mittel für die Parkinsonsche Krankheit

Agents thérapeutiques pour la maladie de Parkinson

(84) Designated Contracting States:
**AT BE CH DE DK ES FR GB GR IE IT LI LU MC NL PT SE**

(30) Priority: **28.09.1992 JP 25783492**

(43) Date of publication of application:
**06.04.1994 Bulletin 1994/14**

(73) Proprietor: **KYOWA HAKKO KOGYO CO., Ltd.**
**Chiyoda-ku, Tokyo 100 (JP)**

(72) Inventors:
- **Suzuki, Fumio, c/o Kyowa Hakko Kogyo Co., Ltd. Tokyo (JP)**
- **Shimada, Junichi, c/o Kyowa Hakko Kogyo Co., Ltd. Tokyo (JP)**
- **Koike, Nobuaki, c/o Kyowa Hakko Kogyo Co., Ltd. Tokyo (JP)**
- **Nakamura, Joji, c/o Kyowa Hakko Kogyo Co., Ltd. Tokyo (JP)**
- **Shiozaki, Shizuo, c/o Kyowa Hakko Kogyo Co., Ltd. Tokyo (JP)**
- **Ichikawa, Shunji, c/o Kyowa Hakko Kogyo Co., Ltd. Tokyo (JP)**
- **Nonaka, Hiromi, c/o Kyowa Hakko Kogyo Co., Ltd. Tokyo (JP)**

(74) Representative: **Lambert, Hugh Richmond et al**
**D. YOUNG & CO.,**
**21 New Fetter Lane**
**London EC4A 1DA (GB)**

(56) References cited:

| | |
|---|---|
| EP-A- 0 092 398 | EP-A- 0 103 497 |
| EP-A- 0 203 721 | EP-A- 0 215 736 |
| EP-A- 0 267 607 | EP-A- 0 374 808 |
| EP-A- 0 415 456 | EP-A- 0 470 317 |
| WO-A-86/02551 | WO-A-92/06976 |
| CH-A- 512 486 | FR-A- 2 514 003 |
| GB-A- 2 135 311 | US-A- 2 729 642 |
| US-A- 3 624 215 | US-A- 3 624 216 |
| US-A- 3 900 474 | US-A- 4 755 517 |
| US-A- 4 968 672 | |

- **CHEMICAL ABSTRACTS, vol. 118, no. 25, 1993, Columbus, Ohio, US; abstract no. 254617s,**
- **J. MED. CHEM. vol. 34, no. 4, 1991, WASHINGTON US pages 1431 - 1435 R.H. ERICKSON ET AL.**
- **DATABASE WPI Week 9303, Derwent Publications Ltd., London, GB; AN 93-021440**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 590 919 B1

## Description

The present invention relates to therapeutic agents for the treatment of Parkinson's disease.

It is known that adenosine exhibits neurotransmitter depressing activity, bronchospasmic activity, and bone absorption promoting activity via an $A_2$ receptor. Therefore, adenosine $A_2$ receptor antagonists (hereinafter referred to as $A_2$-antagonists) are expected as therapeutic agents for various kinds of diseases through activated adenosine $A_2$ receptors, for example, therapeutic agents for Parkinson's disease, anti-dementia agents, anti-asthmatic agents, antidepressants, and therapeutic agents for osteoporosis.

Known compounds in this general field include compounds of formulae A and B:

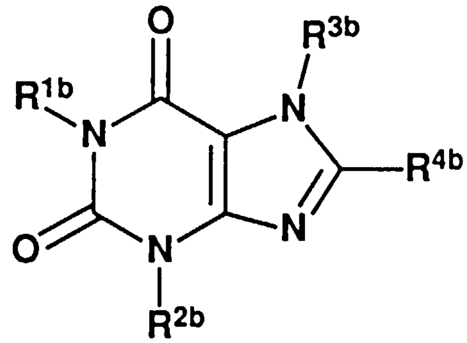

(A)

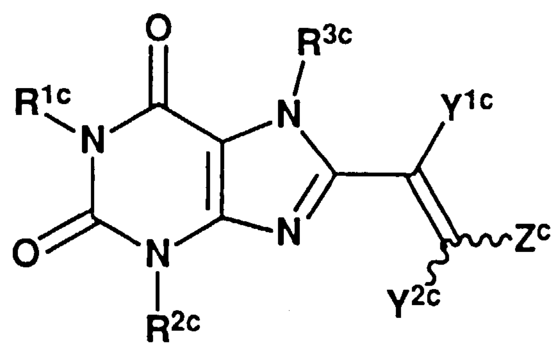

(B)

For example, it is known that adenosine antagonistic activity is found in compounds represented by Formula (A) in which $R^{1b}$ and $R^{2b}$ represent propyl, $R^{3b}$ represents hydrogen, and $R^{4b}$ represents substituted or unsubstituted phenyl, aromatic heterocyclic group, cycloalkyl, styryl, or phenylethyl [J. Med. Chem., 34, 1431 (1991)]. Further, U.S. P. 3,641,010 discloses, as brain stimulants, compounds represented by Formula (B) in which $R^{1c}$ and $R^{2c}$ independently represent methyl or ethyl, $R^{3c}$ represents methyl, $Y^{1c}$ and $Y^{2c}$ represent hydrogen, and $Z^c$ represents phenyl or 3,4,5-trimethoxyphenyl. W092/06976 discloses, as compounds having an adenosine $A_2$ receptor antagonistic activity and therapeutic effects for asthma and osteoporosis, compounds represented by Formula (B) in which $R^{1c}$ and $R^{2c}$ independently represent hydrogen, propyl, butyl, or allyl, $R^{3c}$ represents hydrogen or lower alkyl, $Y^{1c}$ and $Y^{2c}$ independently represent hydrogen or methyl, and $Z^c$ represents phenyl, pyridyl, imidazolyl, furyl, or thienyl unsubstituted or substituted by 1 to 3 substituents such as lower alkyl, hydroxy, lower alkoxy, halogen, amino, and nitro. Furthermore, other compounds represented by Formula (B) are known. One is 8-styryl caffeine which is a compound of Formula (B) in which $R^{1c}$, $R^{2c}$, and $R^{3c}$ represent methyl, $Y^{1c}$ and $Y^{2c}$ represent hydrogen, and $Z^c$ represents phenyl [Chem. Ber. 119, 1525 (1986)]. Another is a compound of Formula (B) in which $R^{1c}$, $R^{2c}$, and $R^{3c}$ represent methyl, $Y^{1c}$ and $Y^{2c}$ represent hydrogen, and $Z^c$ represents pyridyl, quinolyl, or methoxy-substituted or unsubstituted benzothiazolyl [Chem. Abst. 60, 1741h (1964)]. However, there is no description with regard to the pharmacological activity of any of these compounds.

EP-A-0 374 808 discloses xanthines having the structure of the general formula

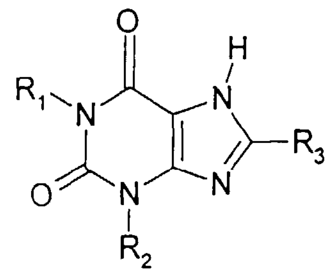

EP-A-0 374 808 teaches that one of the possible indications for these compounds is Parkinson's disease.

US-A-3,641,010 discloses compounds of the following formula

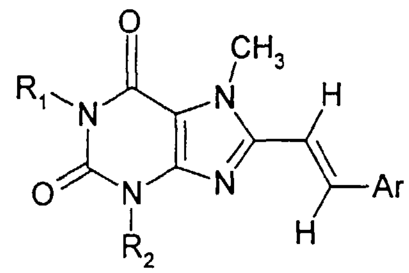

EP-A-0 559 893 relates to xanthine derivatives of the formula

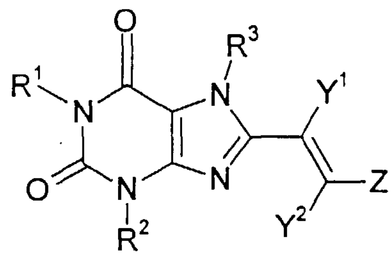

Z represents a substituted or unsubstituted phenyl, pyridyl group, imidazolyl group, furyl group or thienyl group.

EP-A-0 565 377 discloses therapeutic agents for use in treatment of Parkinson's disease, such agents being xanthine derivatives of the following formula

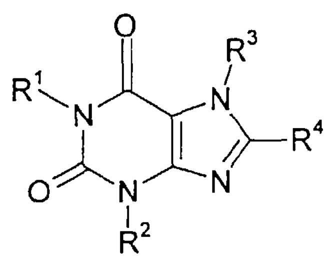

In accordance with the present invention, it has been found that xanthine derivatives of formula I have potent and selective adenosine $A_2$ receptor antagonistic activity and are therefore excellent prospective therapeutic agents for the treatment of Parkinson's disease.

In one aspect, therefore the present invention provides use of a xanthine derivative of Formula (I):

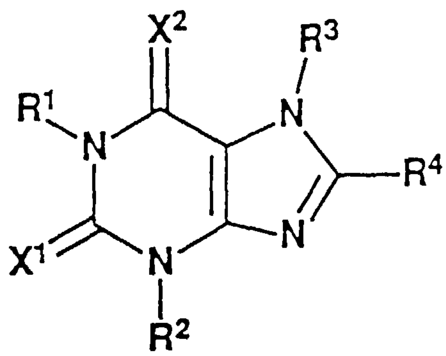

(I)

in which:

$R^1$ and $R^2$ are independently methyl or ethyl;
$R^3$ is hydrogen, $C_1$-$C_6$ straight or branched chain alkyl
$R^4$ is

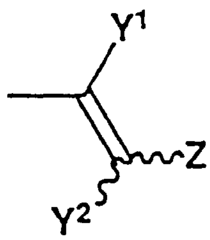

group where $Y^1$ and $Y^2$ are hydrogen, and Z represents a

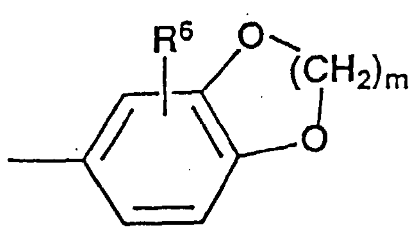

group in which $R^6$ is hydrogen, and m represents 1 or 2;
and $X^1$ and $X^2$ are O;

or a pharmaceutically acceptable salt thereof, for the manufacture of a medicament for the treatment ofParkinson's disease.

The compounds represented by Formula (I) are hereinafter referred to as Compounds (I), and the same applies to the compounds of other formula numbers.

The present invention also provides xanthine derivatives of the formula (I-A):

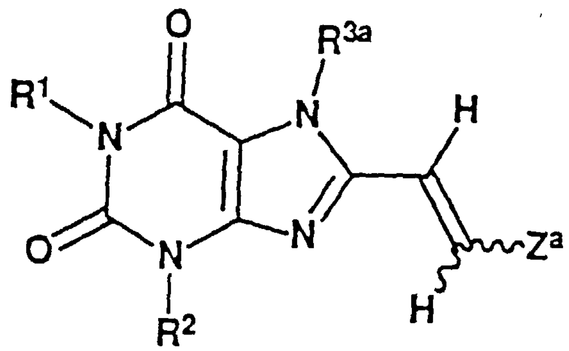

(I-A)

in which:

$R^1$ and $R^2$ are as defined above;
$R^{3a}$ is hydrogen or $C_1$-$C_6$ straight or branched chain alkyl; and
$Z^a$ is a

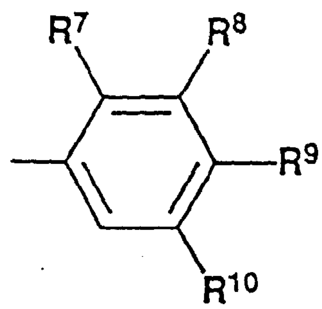

group wherein at least one of $R^7$, $R^8$ and $R^9$ groups is a $C_1$-$C_6$ straight or branched chain alkyl or alkoxy group and the others are hydrogen; and $R^{10}$ is hydrogen or $C_1$-$C_6$ straight or branched chain alkyl; or a

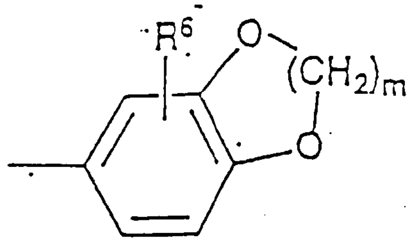

group wherein $R^6$ and m are as defined above;

or a pharmaceutically acceptable salt thereof.

In the descriptive portion of the specification in respect of groups in Formula (I) and Formula (I-A), the lower alkyl and the lower alkyl moiety of the lower alkoxy mean a straight-chain or branched alkyl group having 1 to 6 carbon atoms, such as methyl, ethyl, propyl, isopropyl, butyl, isobutyl, sec-butyl, tert-butyl, pentyl, neopentyl, and hexyl. The lower alkenyl means a straight-chain or branched alkenyl group having 2 to 6 carbon atoms, such as vinyl, allyl, methacryl, crotyl, 3-butenyl, 2-pentenyl, 4-pentenyl, 2-hexenyl, 5-hexenyl. The lower alkynyl means a straight-chain or branched alkynyl group having 2 to 6 carbon atoms, such as ethynyl, propargyl, 2-butynyl, 3-butynyl, 2-pentynyl, 4-pentynyl, 2-hexynyl, 5-hexynyl, 4-methyl-2-pentynyl. The aryl means phenyl or napthyl. The cycloalkyl means a cycloalkyl group having 3 to 8 carbon atoms, such as cyclopropyl, cyclobutyl, cyclopentyl, cyclohexyl, cycloheptyl, and cyclooctyl. Examples of the heterocyclic group are furyl, thienyl, pyrrolyl, pyranyl, thiopyranyl, pyridyl, thiazolyl, imidazolyl, pyrimidyl, triazinyl, indolyl, quinolyl, purinyl, and benzothiazolyl. The halogen includes fluorine, chlorine, bromine, and iodine.

The substituted aryl and the substituted heterocyclic ring each has 1 to 4 independently-selected substituents.

Examples of the substituents are lower alkyl, hydroxy, substituted or unsubstituted lower alkoxy, halogen, nitro, amino, lower alkylamino, di (lower alkyl) amino, trifluoromethyl, trifluoromethoxy, benzyloxy, phenyl, and phenoxy. The lower alkyl and the alkyl moiety of the lower alkoxy, lower alkylamino, and di(lower alkyl)amino have the same meaning as the lower alkyl defined above. The halogen has the same meaning as the halogen defined above. Examples of the substituent of the substituted lower alkoxy are hydroxy, lower-alkoxy, halogen, amino, azide, carboxy, and lower alkoxycarbonyl. The lower alkyl moiety of the lower alkoxy and lower alkoxycarbonyl has the same meaning as the lower alkyl defined above, and the halogen has the same meaning as the halogen defined above.

The above-mentioned pharmaceutically acceptable salts of Compounds (I) include pharmaceutically acceptable acid addition salts, metal salts, ammonium salts, organic amine addition salts, and amino acid addition salts.

Examples of the pharmaceutically acceptable acid addition salts are inorganic acid addition salts such as hydrochloride, sulfate, and phosphate, and organic acid addition salts such as acetate, maleate, fumarate, tartrate, and citrate. Examples of the pharmaceutically acceptable metal salts are alkali metal salts such as sodium salt and potassium salt, alkaline earth metal salts such as magnesium salt and calcium salt, aluminium salt, and zinc salt. Examples of the pharmaceutically acceptable ammonium salts are ammonium salt and tetramethyl ammonium salt. Examples of the pharmaceutically acceptable organic amine addition salts are salts with morpholine and piperidine. Examples of the pharmaceutically acceptable amino acid addition salts are salts with lysine, glycine, and phenylalanine.

The processes for producing Compounds (I) are described below. Compounds (I) can also be produced according to the methods described in, for example, U.S.P. 3,641,010; J. Med. Chem., 34, 1431 (1991); Chem. Ber., 119, 1525 (1986); and Chem. Abst., 60, 1741h (1964).

Process 1

Compound (I-a) [Compound (I) in which $R^3$ is hydrogen] can be prepared by the following reaction steps:

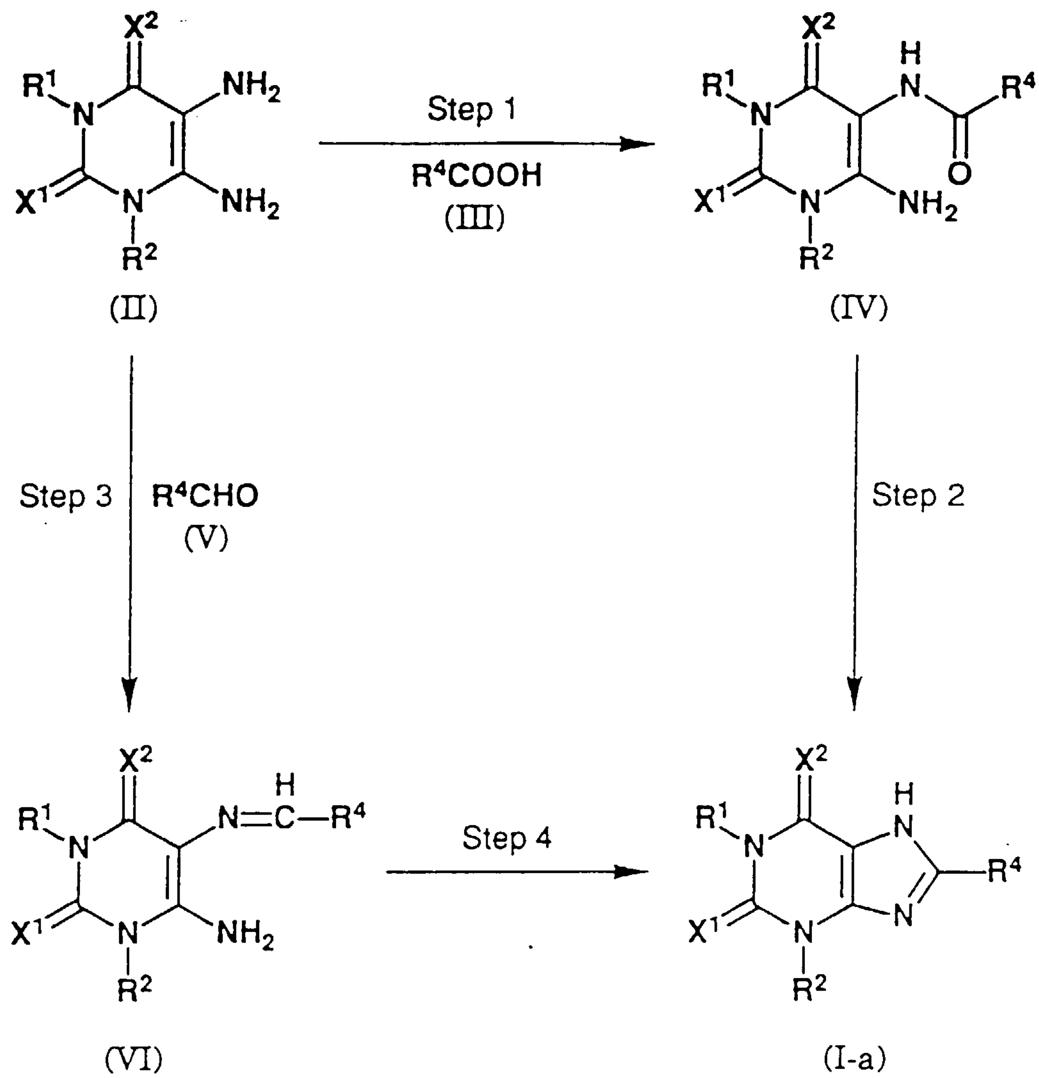


(II) (IV) (VI) (I-a)

(In the formulae, $R^1$, $R^2$, $R^4$, $X^1$, and $X^2$ have the same meanings as defined above.)

(STEP 1)

A uracil derivative (II) obtained by a known method [for example, Japanese Published Unexamined Patent Application No. 42383/84; J. Med. Chem., 32, 1873 (1989)] is allowed to react with either a carboxylic acid (III) or a reactive derivative thereof to give Compound (IV). Examples of the reactive derivative of the carboxylic acid (III) are acid halides such as acid chloride and acid bromide, active esters such as p-nitrophenyl ester and N-oxysuccinimide ester, commercially available acid anhydrides, acid anhydrides produced by using carbodiimides such as 3-(3-dimethylaminopropyl)-1-ethylcarbodiimide, diisopropyl carbodiimide, and dicyclohexyl carbodiimide, and mixed acid anhydrides with monoethyl carbonate or monoisobutyl carbonate. If the carboxylic acid (III) is used, the reaction is completed in 10 minutes to 5 hours at 50 to 200°C without using a solvent.

If a reactive derivative of the carboxylic acid (III) is used, the reaction can be carried out according to a conventional method employed in peptide chemistry. That is, Compound (II) and a derivative of the carboxylic acid (III) are allowed to react in a solvent, preferably in the presence of an additive or a base, to give Compound (IV). Examples of the solvent are halogenated hydrocarbons such as methylene chloride, chloroform, and ethylene dichloride, ethers such as dioxane and tetrahydrofuran, dimethylformamide, dimethylsulfoxide, and water if necessary. An example of the additive is 1-hydroxybenzotriazole. Examples of the base are pyridine, triethylamine, 4-dimethylaminopyridine, and N-methylmorpholine. The reaction is completed in 0.5 to 24 hours at -80 to 50°C. The reactive derivative may be formed in the reaction system and then used without being isolated.

(STEP 2)

Compound (I-a) can be obtained by reaction of Compound (IV) carried out in any of the following manners: in the presence of a base (Method A); by treatment with a dehydrating agent (Method B); or by heating (Method C). In Method A, the reaction is carried out in a solvent in the presence of a base such as an alkali metal hydroxide (e.g. sodium hydroxide and potassium hydroxide). As the solvent, water, lower alcohols such as methanol and ethanol, ethers such as dioxane and tetrahydrofuran, dimethylformamide, dimethylsulfoxide, and the like may be used alone or in combination. The reaction is completed in 10 minutes to 6 hours at 0 to 180°C.

In Method B, the reaction is carried out in an inert solvent or in the absence of a solvent using a dehydrating agent such as a thionyl halide (e.g. thionyl chloride) and a phosphorus oxyhalide (e.g. phosphorus oxychloride). Examples of the inert solvent are halogenated hydrocarbons such as methylene chloride, chloroform and ethylene dichloride, dimethylformamide, and dimethylsulfoxide. The reaction is completed in 0.5 to 12 hours at 0 to 180°C.

In Method C, the reaction is carried out in a polar solvent such as dimethylformamide, dimethylsulfoxide, and Dowtherm A (Dow Chemicals). The reaction is completed in 10 minutes to 5 hours at 50 to 200°C.

(STEP 3)

Compound (II) is allowed to react with an aldehyde (V) to give a Schiff's base (VI). As a reaction solvent, mixtures of acetic acid and a lower alcohol such as methanol and ethanol may be used. The reaction is completed in 0.5 to 12 hours at -20 to 100°C.

(STEP 4)

Compound (VI) is oxidatively cyclized in an inert solvent in the presence of an oxidizing agent to form Compound (I-a). Examples of the oxidizing agent are oxygen, ferric chloride, cerium (IV) ammonium nitrate, and diethylazodicarboxylate. Examples of the inert solvent are lower alcohols such as methanol and ethanol, halogenated hydrocarbons such as methylene chloride and chloroform, and aromatic hydrocarbons such as toluene, xylene, and nitrobenzene. The reaction is completed in 10 minutes to 12 hours at 0 to 180°C.

Process 2

Compound (I-b) [Compound (I) in which $R^3$ is a group other than hydrogen] can be prepared by the following reaction step.

Compound (I-b) is obtained from Compound (I-a) prepared by Process 1.

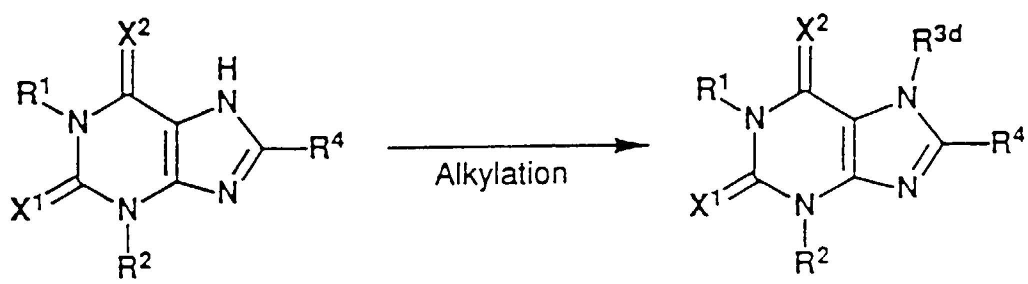


(I-a) (I-b)

(In the formulae, $R^{3d}$ represents a group other than hydrogen in the definition of $R^3$; and $R^1$, $R^2$, $R^4$, $X^1$, and $X^2$ have the same meanings as defined above.)

Compound (I-b) can be obtained by reaction of Compound (I-a) with an alkylating agent, in the presence of a base if necessary. Examples of the alkylating agent are alkyl halides such as methyl iodide, ethyl iodide, and allyl bromide, dialkyl sulfates such as dimethyl sulfate, sulfonic esters such as allyl p-toluenesulfonate and methyl trifluoromethanesulfonate, and diazoalkanes such as diazomethane. Examples of the base are alkali metal carbonates such as sodium carbonate and potassium carbonate, alkali metal hydrides such as sodium hydride, and alkali metal alkoxides such as sodium methoxide and sodium ethoxide. As a reaction solvent, aromatic hydrocarbons such as toluene and xylene, ketones such as acetone and methyl ethyl ketone, dimethylformamide, dimethylsulfoxide, or the like may be used. The reaction is completed in 0.5 to 24 hours at 0 to 180°C.

Process 3 (for subsequent treatment in accordance with Process 8)

Compound (I-d) [Compound (I) in which Z is phenyl having hydroxy as substituent(s)] can be alternatively prepared by the following reaction step.

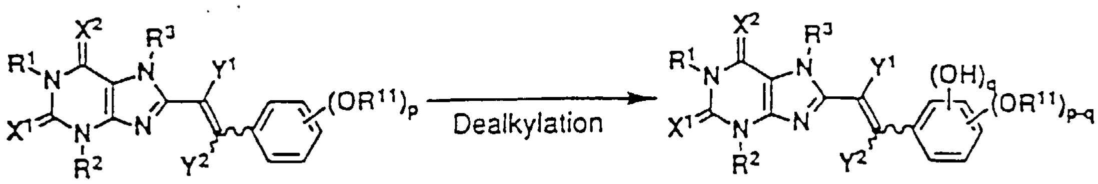


(I-c) (I-d)

(In the formulae, $R^{11}$ represents substituted or unsubstituted lower alkyl; p and q are integers of 1 to 4 and p $\geq$ q; and $R^1$, $R^2$, $R^3$, $X^1$, $X^2$, $Y^1$, and $Y^2$ have the same meanings as defined above.)

The substituted or unsubstituted lower alkyl in the definition of $R^{11}$ has the same meaning as defined above.

Compound (I-d) can be obtained by reaction of Compound (I-c) [Compound (I) in which Z is phenyl having lower alkoxy as substituent(s)] obtained by Process 1 or Process 2 with a dealkylating agent. Examples of the suitable dealkylating agent are boron tribromide and the complex thereof with dimethyl disulfide, boron trichloride, iodotrimethylsilane, sodium ethanethiolate, sodium benzenethiolate, and hydrobromic acid. A reaction solvent is selected from aromatic hydrocarbons such as toluene and xylene, halogenated hydrocarbons such as methylene chloride, chloroform, and ethylene dichloride, dimethylformamide, acetic acid, etc. depending upon the kind of the dealkylating agent used. The reaction is completed in 10 minutes to 120 hours at -30 to 140°C.

Process 4 (for subsequent treatment in accordance with Process 6, 7 or 8)

Compound (I-e) [Compound (I) in which Z is phenyl having lower alkoxy as substituent(s)] can be alternatively prepared by the following reaction step.

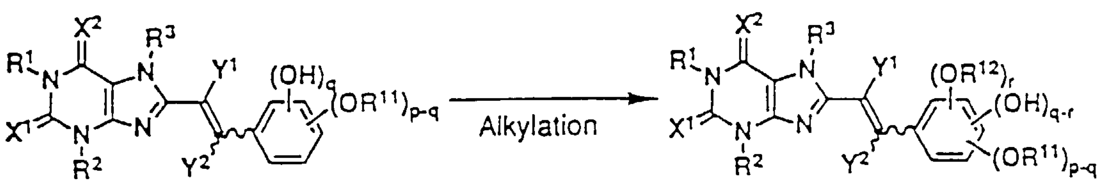


(I-d) (I-e)

(In the formulae, $R^{12}$ represents substituted or unsubstituted lower alkyl; r is an integer of 1 to 4 and q $\geq$ r; and $R^1$, $R^2$, $R^3$, $R^{11}$, $X^1$, $X^2$, $Y^1$, $Y^2$, p, and q have the same meanings as defined above.)

The substituted or unsubstituted lower alkyl in the definition of $R^{12}$ has the same meaning as defined above.

Compound (I-e) can be obtained from Compound (I-d) according to the method of Process 2.

Process 5 (for subsequent treatment in accordance with Process 8)

Compound (I-h) [Compound (I) is which Z phenyl having amino-substituted lower alkoxy as the substituent] can be alternatively prepared by the following reaction step.

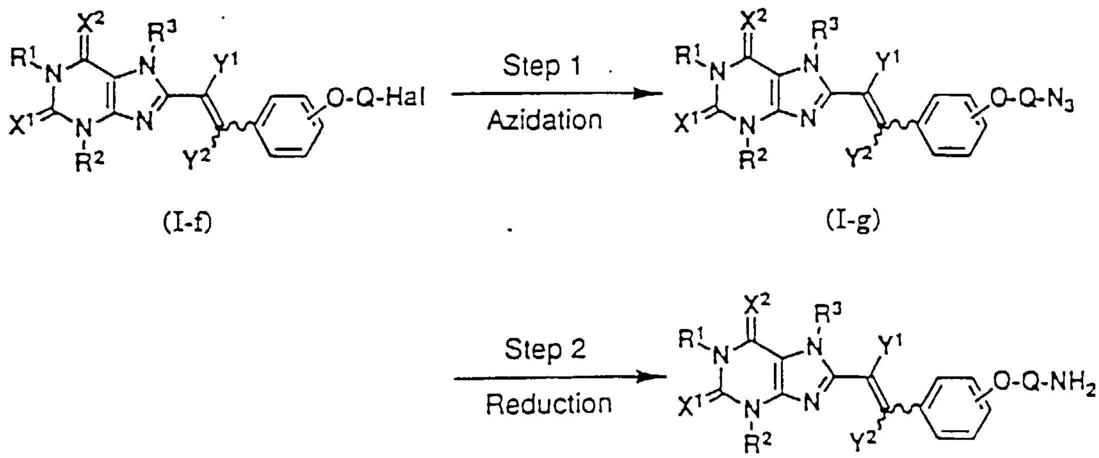


(I-f) (I-g)

(I-h)

(In the formulae, Q represents lower alkylene; Hal represents chlorine, bromine, or iodine; and $R^1$, $R^2$, $R^3$, $X^1$, $X^2$, $Y^1$, and $Y^2$ have the same meanings as defined above.)

The lower alkylene in the definition of Q means a straight-chain or branched alkylene group having 1 to 6 carbon atoms, such as methylene, ethylene, propylene, 1-methylethylene, butylene, 1-methylpropylene, 2-methylpropylene, pentylene, and hexylene.

(STEP 1)

Compound (I-g) can be obtained by reaction of Compound (I-f) [Compound (I) in which Z is phenyl having chlorine, bromine, or iodine-substituted lower alkoxy as the substituent] obtained by Process 4 with 5 to 10 equivalents of sodium azide. As a reaction solvent, an inert solvent such as dimethylformamide may be used. The reaction is completed in 1 to 10 hours at 50 to 80°C.

(STEP 2)

Compound (I-h) can be obtained by treatment of Compound (I-g) in an inert solvent such as tetrahydrofuran and dioxane in the presence of 2 to 5 equivalents of a reducing agent such as triphenylphosphine, followed by addition of an excess of water and further treatment for 1 to 10 hours at 50°C to the boiling point of the solvent used.

<u>Process 6</u> (for subsequent treatment in accordance with Process 8)

Compound (I-j) [Compound (I) in which Z is phenyl having carboxy-subsituted lower alkoxy as the substituent] can be alternatively prepared by the following reaction step.

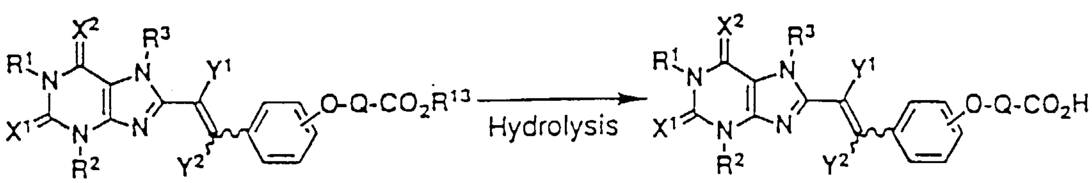


(I-i) (I-j)

(In the formulae, $R^{13}$ represents lower alkyl; and $R^1$, $R^2$, $R^3$, Q, $X^1$, $X^2$, $Y^1$, and $Y^2$ have the same meanings as defined above.)

The lower alkyl in the definition of $R^{13}$ has the same meaning as defined above.

Compound (I-j) can be obtained by hydrolysis of Compound (I-i) [Compound (I) in which Z is phenyl having lower alkoxycarbonyl-substituted lower alkoxy as the substituent] obtained by Process 4 in the presence of an alkali

metal hydroxide such as sodium hydroxide and lithium hydroxide. As a reaction solvent, a mixture of water and an ether such as dioxane and tetrahydrofuran, or a mixture of water and an alcohol such as methanol and ethanol may be used. The reaction is completed in 10 minutes to 12 hours at room temperature to the boiling point of the solvent used.

Process 7 (for subsequent treatment in accordance with Process 8)

Compound (I-m) [Compound (I) in which Z is phenyl having hydroxy as the substituent(s)] can be alternatively prepared by the following reaction step.

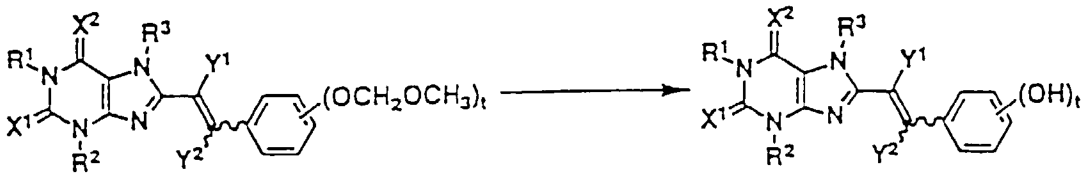

(I-k) (I-m)

(In the formulae, t is an integer of 1 to 4; and $R^1$, $R^2$, $R^3$, $X^1$, $X^2$, $Y^1$, and $Y^2$ have the same meanings as defined above.)

Compound (I-m) can be obtained by treatment of Compound (I-k) [Compound (I) in which Z is phenyl having methoxymethoxy as the substituent(s)] obtained by Process 1, Process 2, or Process 4 in the presence of hydrogen chloride gas, an aqueous solution of hydrochloric acid, or the like. As a reaction solvent, ethers such as dioxane and tetrahydrofuran, alcohols such as methanol and ethanol, or the like may be used. The reaction is completed in 1 to 20 hours at room temperature to the boiling point of the solvent used.

Process 8

Compound (I-o) [Compound (I) in which $X^2$ is S] can be alternatively prepared by the following reaction step.

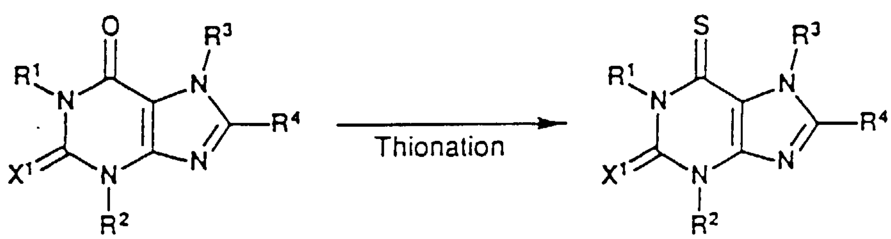


(I-n) (I-o)

(In the formulae, $R^1$, $R^2$, $R^3$, $R^4$, and $X^1$ have the same meanings as defined above.)

Compound (I-o) can be obtained by reaction of Compound (I-n) [Compound (I) in which $X^2$ is 0] obtained by Process 1 to Process 7 with a thionating agent. Examples of the thionating agent are phosphorus pentachloride and Lawesson's reagent. As a reaction solvent, pyridine, dimethylformamide, dioxane, tetrahydrofuran, or the like, preferably pyridine, may be used. The reaction is completed in 10 minutes to 36 hours at 50 to 180°C.

The desired compounds in the processes described above can be isolated and purified by purification methods conventionally used in organic synthetic chemistry, for example, filtration, extraction, washing, drying, concentration, recrystallization, and various kinds of chromatography.

In the case where a salt of Compound (I) is desired and it is produced in the form of the desired salt, it can be subjected to purification as such. In the case where Compound (I) is produced in the free state and its salt is desired, Compound (I) is dissolved or suspended in a suitable solvent, followed by addition of an acid or a base to form a salt.

Some of Compounds (I) can exist in the form of geometrical isomers such as an (E)-isomer and a (Z)-isomer, and the present invention covers all possible isomers including the above-mentioned ones and mixtures thereof. In the case where separation between an (E)-isomer and a (Z)-isomer is desired, they can be isolated and purified by fractionation methods, for example, fractional crystallization, fractional precipitation, and fractional dissolution.

Compounds (I) and pharmaceutically acceptable salts thereof may be in the form of adducts with water or various solvents, which can also be used as the therapeutic agents of the present invention.

Examples of Compounds (I) are shown in Table 1. Compounds numbered 49 to 66 and 69 to 80 are intentionally omitted from Table 1.

## Table 1-1

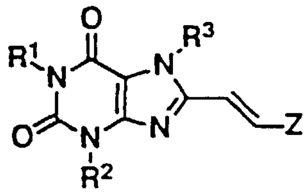

| Compound | $-R^1$ | $-R^2$ | $-Z$ | $-R^3$ |
|---|---|---|---|---|
| 1 | $-CH_2CH_3$ | $-CH_2CH_3$ | 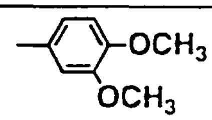 | $-H$ |
| 2 | " | " | " | $-CH_3$ |
| 3 | " | " | 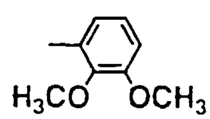 | $-H$ |
| 4 | " | " | " | $-CH_3$ |
| 5 | " | " | 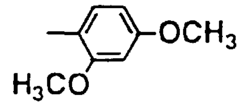 | $-H$ |
| 6 | " | " | " | $-CH_3$ |
| 7 | " | " | 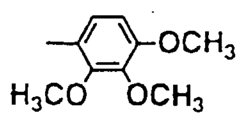 | $-H$ |
| 8 | " | " | " | $-CH_3$ |
| 9 | " | " | 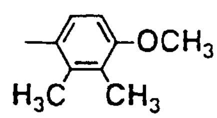 | $-H$ |
| 10 | " | " | " | $-CH_3$ |
| 11 | " | " | 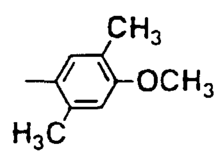 | $-H$ |
| 12 | " | " | " | $-CH_3$ |
| 13 | " | " | 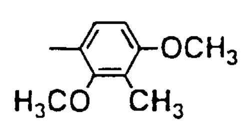 | $-H$ |
| 14 | " | " | " | $-CH_3$ |

Table 1-2

| Compound | $-R^1$ | $-R^2$ | $-Z$ | $-R^3$ |
|---|---|---|---|---|
| 15 | $-CH_2CH_3$ | $-CH_2CH_3$ | 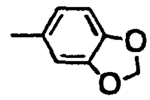 | $-H$ |
| 16 | " | " | " | $-CH_3$ |
| 17 | " | " | 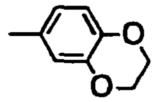 | $-H$ |
| 18 | " | " | " | $-CH_3$ |
| 19 | $-CH_3$ | $-CH_3$ | 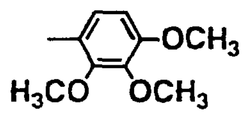 | $-H$ |
| 20 | " | " | " | $-CH_3$ |
| 21 | " | " | 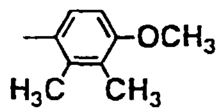 | $-H$ |
| 22 | " | " | " | $-CH_3$ |
| 23 | " | " | 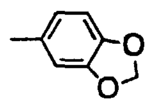 | $-H$ |
| 24 | " | " | " | $-CH_3$ |
| 25 | " | " | 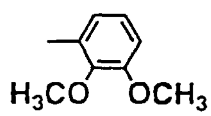 | $-H$ |
| 26 | " | " | " | $-CH_3$ |
| 27 | " | " | 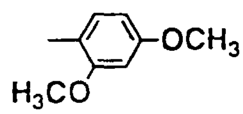 | $-H$ |
| 28 | " | " | " | $-CH_3$ |
| 29 | " | " | 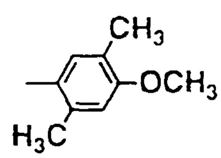 | $-H$ |
| 30 | " | " | " | $-CH_3$ |
| 31 | " | " | 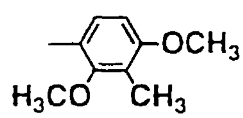 | $-H$ |
| 32 | " | " | " | $-CH_3$ |

Table 1-3

| Compound | $-R^1$ | $-R^2$ | $-Z$ | $-R^3$ |
|---|---|---|---|---|
| 33 | $-CH_2CH_3$ | $-CH_2CH_3$ | 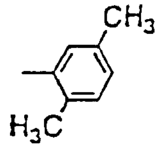 | $-H$ |
| 34 | " | " | " | $-CH_3$ |
| 35 | " | " | 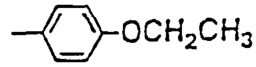 | $-H$ |
| 36 | " | " | " | $-CH_3$ |
| 37 | " | " | 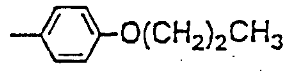 | $-H$ |
| 38 | " | " | " | $-CH_3$ |
| 39 | " | " | 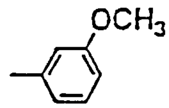 | $-H$ |
| 40 | " | " | " | $-CH_3$ |
| 41 | " | " | 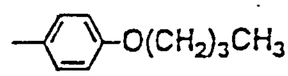 | $-H$ |
| 42 | " | " | " | $-CH_3$ |
| 43 | " | " | 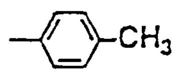 | $-H$ |
| 44 | " | " | " | $-CH_3$ |
| 45 | " | " | 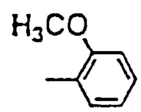 | $-H$ |
| 46 | " | " | " | $-CH_3$ |
| 47 | " | " | 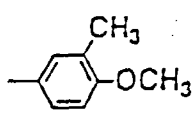 | $-H$ |
| 48 | " | " | " | $-CH_3$ |

Table 1-4

| Compound | $-R^1$ | $-R^2$ | $-Z$ | $-R^3$ |
|---|---|---|---|---|
| 67 | " | " | 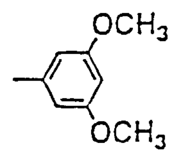 | $-H$ |
| 68 | " | " | " | $-CH_3$ |

Table 1-5

| Compound | $-R^1$ | $-R^2$ | $-Z$ | $-R^3$ |
|---|---|---|---|---|
| 81 | " | " | 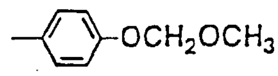 | $-H$ |
| 82 | " | " | " | $-CH_3$ |

The pharmacological activities of Compounds (I) are shown below by experimental examples.

Experimental Example 1 Acute Toxicity Test

Test compounds were orally administered to groups of dd-strain male mice weighing 20 ± 1 g, each group consisting of three mice. Seven days after the administration, minimum lethal dose (MLD) of each compound was determined by observing the mortality.

The results are shown in Table 2.

Table 2-1

| Compound | MLD(mg/kg) | Compound | MLD(mg/kg) |
|---|---|---|---|
| 1 | > 300 | 31 | > 100 |
| 2 | > 300 | 32 | > 100 |
| 3 | > 300 | 33 | > 300 |
| 4 | > 300 | 34 | > 300 |
| 5 | > 100 | 35 | > 100 |
| 6 | > 300 | 36 | > 300 |
| 7 | > 300 | 37 | > 100 |
| 8 | > 300 | 38 | > 300 |
| 9 | > 300 | 39 | > 100 |
| 10 | > 300 | 40 | > 300 |
| 11 | > 300 | 41 | > 100 |
| 12 | > 300 | 42 | > 100 |

Table 2-1 (continued)

| Compound | MLD(mg/kg) | Compound | MLD(mg/kg) |
|---|---|---|---|
| 13 | > 300 | 43 | > 100 |
| 14 | > 300 | 44 | > 100 |
| 15 | > 300 | 45 | > 100 |
| 16 | > 300 | 46 | > 100 |
| 17 | > 100 | 47 | > 100 |
| 18 | > 300 | 48 | > 100 |
| 19 | > 300 | | |
| 20 | > 300 | | |
| 21 | > 100 | | |
| 22 | > 100 | | |
| 23 | > 300 | | |
| 24 | > 300 | | |
| 25 | > 100 | | |
| 26 | > 300 | | |
| 27 | > 100 | | |
| 28 | > 300 | | |
| 29 | > 100 | | |
| 30 | > 100 | | |

Table 2-2

| Compound | MLD(mg/kg) |
|---|---|
| 67 | > 100 |
| 68 | > 300 |
| 81 | > 100 |
| 82 | > 100 |

As shown in Table 2, the MLD values of all the compounds are greater than 100 mg/kg or 300 mg/kg, indicating that the toxicity of the compounds is weak. Therefore, these compounds can be safely used in a wide range of doses.

Experimental Example 2 Adenosine Receptor Antagonistic Activity

1) Adenosine $A_1$ Receptor Binding Test

The test was conducted according to the method of Bruns et al. [Proc. Natl. Acad. Sci., 77, 5547 (1980)] with slight modification.

Cerebrum of a guinea pig was suspended in ice-cooled 50 mM tris(hydroxymethyl)aminomethane hydrochloride (Tris HCl) buffer (pH 7.7) by using Polytron homogenizer (manufactured by Kinematicas Co.). The suspension was centrifuged (50,000 x g, 10 minutes), and the precipitate was suspended again in the same amount of 50 mM Tris HCl buffer. The suspension was centrifuged under the same conditions, and the final precipitate was suspended once again in 50 mM Tris HCl buffer to give a tissue concentration of 100 mg (wet weight)/ml. The tissue suspension was incubated at 37°C for 30 minutes in the presence of 0.02 unit/mg tissue of adenosine deaminase (manufactured by Sigma Co.). The tissue suspension was then centrifuged (50,000 x g, 10 minutes), and 50 mM Tris HCl buffer was added to the precipitate to adjust the concentration of tissue to 10 mg (wet weight)/ml.

To 1 ml of the tissue suspension thus prepared were added 50 µl of cyclohexyladenosine labeled with tritium ($^3$H-CHA: 27 Ci/mmol, manufactured by New England Nuclear Co.) (final concentration: 1.1 nM) and 50 µl of a test compound. The mixture solution was allowed to stand at 25°C for 90 minutes and then rapidly filtered by suction through a glass fiber filter (GF/C manufactured by Whatman Co.). The filter was immediately washed three times with 5 ml each of ice-cooled 50 mM Tris HCl buffer, and transferred to a vial, and a scintillator (EX-H by Wako Pure Chemical Industries, Ltd.) was added thereto. The radioactivity on the filter was determined with a liquid scintillation counter (manufactured by Packard Instrument Co.).

The inhibition rate of the test compound against the binding of $A_1$ receptor ($^3$H-CHA binding) was calculated

by the following equation:

$$\text{Inhibition Rate (\%)} = \left(1 - \frac{[B] - [N]}{[T] - [N]}\right) \times 100$$

[Notes]

1. "B" means the amount of radioactivity of $^3$H-CHA bound in the presence of a test compound at a concentration shown in Table 3.
2. "T" means the amount of radioactivity of $^3$H-CHA bound in the absence of a test compound.
3. "N" means the amount of radioactivity of $^3$H-CHA bound in the presence of 10 μM $N^6$-(L-2-phenylisopropyl) adenosine (manufactured by Sigma Co.).

The results are shown in Table 3. The inhibition constant (Ki value) shown in the table was calculated by the Cheng-Prusoff's equation.

Table 3

| | $A_1$ Receptor | | |
|---|---|---|---|
| Compound | Inhibition (%) | | $K_i$(nM) |
| | $10^{-5}$M | $10^{-4}$M | |
| 1 | 84 | 86 | |
| 2 | 67 | 62 | |
| 4 | 55 | 70 | |
| 7 | 34 | 49 | > 10,000 |
| 8 | 89 | 94 | |
| 9 | 14 | 26 | >100,000 |
| 10 | 57 | 56 | |
| 13 | 59 | 71 | |
| 14 | 84 | 86 | |
| 15 | 25 | 35 | >100,000 |
| 16 | 53 | 72 | |
| 17 | 23 | 31 | >100,000 |
| 18 | 56 | 66 | |
| 19 | 10 | 31 | >100,000 |
| 20 | 11 | 1 | >100,000 |
| 21 | 36 | 40 | >100,000 |
| 22 | 1 | 1 | >100,000 |
| 23 | 32 | 38 | >100,000 |
| 24 | -4 | -25 | >100,000 |

2) Adenosine $A_2$ Receptor Binding test

The test was conducted according to the method of Bruns et al. [Mol. Pharmacol., 29, 331 (1986)] with slight modification.

The similar procedure as in the above-described adenosine $A_1$ receptor binding test was repeated using rat corpus striatum to obtain the final precipitate of the tissue thereof. The precipitate was suspended in 50 mM Tris HCl buffer containing 10 mM magnesium chloride and 0.02 unit/mg tissue of adenosine deaminase (manufactured by Sigma Co.) to give a tissue concentration of 5 mg (wet weight)/ml.

To 1 ml of the tissue suspension thus prepared were added 50 μl of a mixture of N-ethylcarboxamidoadenosine labeled with tritium ($^3$H-NECA: 26 Ci/mmol, manufactured by Amersham Co.) (final concentration: 3.8 nM) and cyclopentyladenosine (CPA, manufactured by Sigma Co.) (final concentration: 50 nM), and 50 μl of a test compound.

The mixture solution was allowed to stand at 25°C for 120 minutes and then treated in the same manner as in the adenosine $A_1$ receptor binding test to determine the radioactivity bound to the $A_2$ receptors.

The inhibition rate of the test compound against the binding of $A_2$ receptor ($^3$H-NECA binding) was calculated by the following equation:

$$\text{Inhibition Rate (\%)} = \left(1 - \frac{[B] - [N]}{[T] - [N]}\right) \times 100$$

[Notes]

1. "B" means the amount of radioactivity of $^3$H-NECA bound in the presence of a test compound at a concentration shown in Table 4.
2. "T" means the amount of radioactivity of $^3$H-NECA bound in the absence of a test compound.
3. "N" means the amount of radioactivity of $^3$H-NECA bound in the presence of 100 μM CPA.

The similar procedure as above was repeated to determine the radioactivity bound to the $A_2$ receptors using 50 μl of CGS 21680 labeled with tritium [$^3$H-2-[p-(2-carboxyethyl)-phenethylamino]-5'-(N-ethylcarboxamide)adenosine: 40 Ci/mmol, manufactured by New England Nuclear Co. (J. Pharmacol. Exp. Ther., 251, 888 (1989)] (final concentration: 4.0 nM) in place of 50 μl of the mixture of N-ethylcarboxamidoadenosine labeled with tritium ($^3$H-NECA: 26 Ci/mmol, manufactured by Amersham Co.) (final concentration: 3.8 nM) and cyclopentyladenosine (CPA, manufactured by Sigma Co.) (final concentration: 50 nM).

The inhibition rate of the test compound against the binding of $A_2$ receptors ($^3$H-CGS 21680 binding) was calculated by the following equation:

$$\text{Inhibition Rate (\%)} = \left(1 - \frac{[B] - [N]}{[T] - [N]}\right) \times 100$$

[Notes]

1. "B" means the amount of radioactivity of $^3$H-CGS 21680 bound in the presence of a test compound at a concentration shown in Table 4.
2. "T" means the amount of radioactivity of $^3$H-CGS 21680 bound in the absence of a test compound.
3. "N" means the amount of radioactivity of $^3$H-CGS 21680 bound in the presence of 100 μM CPA.

The results are shown in Table 4. The Ki value ($^3$H-NECA binding) shown in the table was calculated by the following equation:

$$Ki = \frac{IC_{50}}{1 + \frac{L}{Kd} + \frac{C}{Kc}}$$

[Notes]

$IC_{50}$: Concentration at which the inhibition rate is 50%
L: Concentration of $^3$H-NECA
Kd: Dissociation constant of $^3$H-NECA
C: Concentration of CPA
Kc: Inhibition constant of CPA

Table 4-1

| Compd. | $A_2$ Receptor | | | | | Ratio ($A_1/A_2$) |
|---|---|---|---|---|---|---|
| | Inhibition (%) | | | | $K_i$ (nM) | |
| | $10^{-7}$M | $10^{-6}$M | $10^{-5}$M | $10^{-4}$M | | |
| 1 | 53 | 86 | 98 | 100 | 23 | |
| 2 | 67 | 79 | 94 | 92 | 12 | |
| 4 | 57 | 84 | 88 | 90 | 33 | |
| 7 | 65 | 88 | 75 | 83 | 15 | > 670 |
| 8 | 80 | 99 | 95 | 74 | 3.9 | |
| 9 | 79 | 84 | 73 | 86 | 3.1 | > 32,000 |
| 10 | 91 | 94 | 90 | 93 | 2.0 | |
| 13 | 77 | 84 | 88 | 95 | 9.5 | |
| 14 | 85 | 91 | 96 | 98 | 1.6 | |
| 15 | 53 | 64 | 70 | 70 | 15 | |
| 16 | 80 | 96 | 88 | 93 | 6.1 | |
| 17 | 70 | 69 | 80 | 80 | 6.3 | > 16,000 |
| 18 | 84 | 90 | 104 | 98 | 1.5 | |
| 19 | 65 | 87 | 92 | 91 | 310 | > 320 |
| 20 | 51 | 83 | 90 | 88 | 50 | > 2,000 |
| 21 | 56 | 68 | 83 | 79 | 22 | |
| 22 | 78 | 85 | 78 | 81 | 3.0 | > 33,000 |
| 23 | 38 | 60 | 90 | 81 | | |
| 24 | 62 | 57 | 75 | 85 | 26 | > 3,800 |

Table 4-2

| Compd. | $A_2$ Receptor | | | |
|---|---|---|---|---|
| | Inhibition (%) | | | |
| | $10^{-7}$M | $10^{-6}$M | $10^{-5}$M | $10^{-4}$M |
| 25 | | | 76 | 70 |
| 26 | 27 | 59 | 75 | 79 |
| 27 | | | 89 | 90 |
| 28 | 46 | 80 | 86 | 99 |
| 31 | 74 | 82 | 89 | 79 |
| 32 | 83 | 84 | 82 | 98 |
| 33 | | | 93 | 92 |
| 34 | 86 | 93 | 90 | 94 |

Table 4-3

| Compd. | $A_2$ Receptor Inhibition (%) | |
|---|---|---|
| | $10^{-7}$M | $10^{-6}$M |
| 35 | 54 | 71 |
| 36 | 71 | 101 |
| 37 | 56 | 60 |
| 38 | 71 | 94 |
| 39 | 80* | 88* |

*; [$^3$H]CGS 21680 was used.

Table 4-3 (continued)

| Compd. | $A_2$ Receptor Inhibition (%) | |
|---|---|---|
| | $10^{-7}$M | $10^{-6}$M |
| 40 | 88* | 98* |
| 41 | 27* | 52* |
| 42 | 46* | 87* |
| 43 | 68* | 70* |
| 44 | 64* | 103* |
| 47 | 67* | 92* |
| 48 | 83* | 99* |
| 67 | 73 | 73 |
| 68 | 81 | 88 |
| 81 | 78* | 95* |
| 82 | 86* | 98* |

*; [$^3$H]CGS 21680 was used.

As shown in Tables 3 and 4, Compounds (I) and pharmaceutically acceptable salts thereof exhibit an extremely potent affinity especially for adenosine $A_2$ receptors.

Experimental Example 3 Effect on Locomotor Activity in Parkinson's Disease Model in Mice

1-Methyl-4-phenyl-1,2,3,6-tetrahydropyridine (MPTP) is known to cause an acute Parkinson's syndrome (parkinsonism) when administered to humans. The syndrome resembles spontaneous parkinsonism in terms of cardinal symptoms (muscular rigidity, bradycinesia, and resting tremor) and pathological phenomena (extensive degeneration of the nigrostriatal dopamine system) [Science, 219, 979 (1983)]. MPTP-treated mice also exhibit the syndrome similar to parkinsonism [Science, 224, 1451 (1984)].

Especially, MPTP-treated C57BL/6 mice have been reported to serve as a suitable model for Parkinson's disease. In this strain of mice, striatal dopamine is remarkably decreased and locomotor activity is profoundly depressed [Brain Res., 528, 181 (1990)].

The experiment was performed by using several groups of 7-weeks-old male C57BL/6 mice (weighing 20 to 24 g, Japan SLC), each group consisting of 8 mice. MPTP (Aldrich Chemical Co., Inc.) dissolved in a physiological saline solution (Otsuka Pharmaceutical Co., Ltd.) was intraperitoneally administered to each mouse once a day for five consecutive days at a dose of 30 mg/kg. A test compound was suspended in injectable distilled water (Otsuka Pharmaceutical Co., Ltd.) containing Tween 80 [polyoxyethylene (20) sorbitan monooleate]. L-DOPA (Kyowa Hakko Kogyo Co., Ltd.) was suspended in 0.3% CMC (sodium carboxylmethylcellulose) and bromocriptine was suspended in injectable distilled water. Thirty minutes after the final MPTP administration, the test compound suspensions and the control suspension [injectable distilled water (Otsuka Pharmaceutical Co., Ltd.) containing Tween 80] containing no test compound were orally administered to separate groups of the mice (0.1 ml per 10 g of body weight). The amount of active movements (horizontal activity) of each mouse was measured by using Automex-II (Columbus Instruments International Corp.) for the period of 30 minutes starting 30 minutes after the administration of the test compound. Bromocriptine was administered 3 hours prior to the final MPTP treatment, and the amount of active movements was measured for the period of 30 minutes from 1 hour after the MPTP treatment. The effect of the compounds was evaluated by comparing the average counts of the active movements of the test compound-administered groups with those of the control groups. Statistical comparison of the values was carried out by Student's t-test.

The results are shown in Table 5.

Table 5-1

| Group | Administration | Dose of Test Compound (mg/kg) | | Amount of Active Movements (average count ± S.E.M) |
|---|---|---|---|---|
| Normal Control | MPTP<br>Test Compound | (-)<br>(-) | - | 2210 ±101.1 |

Table 5-1 (continued)

| Group | Administration | Dose of Test Compound (mg/kg) | | Amount of Active Movements (average count ± S.E.M) |
|---|---|---|---|---|
| MPTP | MPTP | (+) | | |
| | Test Compound | (-) | - | 45 ± 10.7 ## |
| Compound 2 | MPTP | (+) | | |
| | Compound 2 | (+) | 10 | 637 ±160.0 * |
| Compound 8 | MPTP | (+) | | |
| | Compound 8 | (+) | 10 | 924 ±219.5 ** |

##: $p<0.01$ (comparison with normal control group)

*: $P<0.05$ ; **: $p<0.01$ (comparison with MPTP-treated group)

Table 5-2

| Group | Administration | Dose of Test Compound (mg/kg) | | Amount of Active Movements (average count ± S.E.M) |
|---|---|---|---|---|
| Normal Control | MPTP | (-) | | |
| | Test Compound | (-) | - | 2205 ±232.3 |
| MPTP | MPTP | (+) | | |
| | Test Compound | (-) | - | 60 ± 20.8 ## |
| Compound 2 | MPTP | (+) | | |
| | Compound 2 | (+) | 2.5 | 1265 ±316.9 ** |
| Compound 8 | MPTP | (+) | | |
| | Compound 8 | (+) | 2.5 | 800 ±156.8 ** |

##: $p<0.01$ (comparison with normal control group)

**: $p<0.01$ (comparison with MPTP-treated group)

Table 5-3

| Group | Administration | Dose of Test Compound (mg/kg) | | Amount of Active Movements (average count ± S.E.M) |
|---|---|---|---|---|
| Normal Control | MPTP | (-) | | |
| | Test Compound | (-) | - | 2078 ±180.2 |
| MPTP | MPTP | (+) | | |
| | Test Compound | (-) | - | 132 ± 65.3 ## |
| Compound 2 | MPTP | (+) | | |
| | Compound 2 | (+) | 0.63 | 610 ±147.9 * |

##: $p<0.01$ (comparison with normal control group)

*: $p<0.05$ (comparison with MPTP-treated group)

Table 5-4

| Group | Administration | Dose of Test Compound (mg/kg) | | Amount of Active Movements (average count ± S.E.M) |
|---|---|---|---|---|
| Normal Control | MPTP | (-) | | |
| | Test Compound | (-) | - | 2326 ±147.1 |

Table 5-4 (continued)

| Group | Administration | Dose of Test Compound (mg/kg) | | Amount of Active Movements (average count ± S.E.M) |
|---|---|---|---|---|
| MPTP | MPTP | (+) | | |
| | Test Compound | (-) | - | 71 ± 37.2 ## |
| Compound 10 | MPTP | (+) | | |
| | Compound 10 | (+) | 10 | 754 ±174.2 ** |
| Compound 14 | MPTP | (+) | | |
| | Compound 14 | (+) | 10 | 817 ±163.1 ** |

##: p<0.01 (comparison with normal control group)
**: p<0.01 (comparison with MPTP-treated group)

Table 5-5

| Group | Administration | Dose of Test Compound (mg/kg) | | Amount of Active Movements (average count ± S.E.M) |
|---|---|---|---|---|
| Normal Control | MPTP | (-) | | |
| | Test Compound | (-) | - | 2574 ±165.9 |
| MPTP | MPTP | (+) | | |
| | Test Compound | (-) | - | 21 ± 5.1 ## |
| Compound 34 | MPTP | (+) | | |
| | Compound 34 | (+) | 10 | 157 ± 25.0 ** |

##: p<0.01 (comparison with normal control group)
**: p<0.01 (comparison with MPTP-treated group)

Table 5-6

| Group | Administration | Dose of Test Compound (mg/kg) | | Amount of Active Movements (average count ± S.E.M) |
|---|---|---|---|---|
| Normal Control | MPTP | (-) | | |
| | Test Compound | (-) | - | 2349 ±121.7 |
| MPTP | MPTP | (+) | | |
| | Test Compound | (-) | - | 44 ± 14.4 ## |
| Compound 20 | MPTP | (+) | | |
| | Compound 20 | (+) | 2.5 | 937 ±189.5 ** |

##: p<0.01 (comparison with normal control group)
*: p<0.05 ; **: p<0.01 (comparison with MPTP-treated group)

Table 5-7

| Group | Administration | Dose of Test Compound (mg/kg) | | Amount of Active Movements (average count ± S.E.M) |
|---|---|---|---|---|
| Normal Control | MPTP | (-) | | |
| | Test Compound | (-) | - | 1875 ± 77.7 |
| MPTP | MPTP | (+) | | |
| | Test Compound | (-) | - | 207 ± 85.5 ## |

##: p<0.01 (comparison with normal control group)

Table 5-7 (continued)

| Group | Administration | Dose of Test Compound (mg/kg) | | Amount of Active Movements (average count ± S.E.M) |
|---|---|---|---|---|
| L-DOPA | MPTP | (+) | | |
| | L-DOPA | (+) | 300 | 561 ±271.01 1) |

1): no significant difference as compared with MPTP-treated group)

Table 5-8

| Group | Administration | Dose of Test Compound (mg/kg) | | Amount of Active Movements (average count ± S.E.M) |
|---|---|---|---|---|
| Normal Control | MPTP | (-) | | |
| | Test Compound | (-) | - | 1984 ±122.3 |
| MPTP | MPTP | (+) | | |
| | Test Compound | (-) | - | 41 ± 14.3 ## |
| Bromocriptine | MPTP | (+) | | |
| | Bromocriptine | (+) | 40 | 1739 ±494.9 ** |

##: $p<0.01$ (comparison with normal control group)

**: $p<0.01$ (comparison with MPTP-treated group)

Table 5-9

| Group | Administration | Dose of Test Compound (mg/kg) | | Amount of Active Movements (average count ± S.E.M) |
|---|---|---|---|---|
| Normal Control | MPTP | (-) | | |
| | Test Compound | (-) | - | 2574 ±165.9 |
| MPTP | MPTP | (+) | | |
| | Test Compound | (-) | - | 21 ± 5.1 ## |
| Bromocriptine | MPTP | (+) | | |
| | Bromocriptine | (+) | 10 | 66 ± 35.4 1) |

##: $p<0.01$ (comparison with normal control group)

1) : no significant difference as compared with MPTP-treated group)

As shown in Table 5, L-DOPA (metabolic precursor of dopamine) and bromocriptine (dopamine receptor agonist), which are conventionally used drugs for Parkinson's disease, exhibited only a weak inhibitory activity against the depression of locomotor activity caused by MPTP by oral administration at a dose of 300 and 40 mg/kg, respectively. On the other hand, Compounds (I) showed a potent inhibitory activity at a dose of 10 mg/kg or less than 10 mg/kg.

Experimental Example 4 Effect on Haloperidol-Induced Catalepsy

Parkinson's disease is a clinical syndrome caused by degeneration of nigrostriatal dopaminergic neurons. Systemic administration of haloperidol (dopamine $D_1/D_2$ antagonist) induces catalepsy resulting from the blockage of postsynaptic dopamine $D_2$ receptors. It is generally accepted that this haloperidol-induced catalepsy is a classical model of parkinsonism in humans [Eur. J. Pharmacol., 182, 327 (1990)].

The experiment was performed by using several groups of 5-weeks-old male ddY mice (weighing 22 to 24 g, Japan SLC), each group consisting of 5 mice. Haloperidol (Janssen Pharmaceutica) suspended in 0.3% CMC was intraperitoneally administered to each mouse at a dose of 1.0 mg/kg. Test compounds were suspended in 0.3% CMC or in injectable distilled water (Otsuka Pharmaceutical Co., Ltd.) containing Tween 80. L-DOPA (Kyowa Hakko Kogyo Co., Ltd.) and benserazide hydrochloride (Kyowa Hakko Kogyo Co., Ltd.) were suspended in 0.3% CMC. One hour after the haloperidol administration, the test compound suspensions and the control suspension [injectable distilled water (Otsuka Pharmaceutical Co., Ltd.) containing Tween 80] containing no test compound were orally administered to separate groups of the mice (0.1 ml per 10 g of body weight). One hour after the administration of the test compound,

the forelimbs of each mouse and subsequently the hindlimbs of the same mouse were placed on a 4.5 cm-high, 1.0 cm-wide bar and catalepsy was estimated. All of the test compounds were orally administered at a dose of 10 mg/kg, and L-DOPA (100 mg/kg) and benserazide (25 mg/kg) were intraperitoneally administered together as a control experiment. The catalepsy score and the standard of judgment are shown below.

| score | duration of the cataleptic posture | |
|---|---|---|
| 0: | forelimbs | less than 5 seconds |
| | hindlimbs | less than 5 seconds |
| 1: | forelimbs | from 5 (inclusive) to 10 (exclusive) seconds |
| | hindlimbs | less than 5 seconds |
| 2: | forelimbs | 10 seconds or more |
| | hindlimbs | less than 5 seconds |
| 3: | forelimbs | from 5 (inclusive) to 10 (exclusive) seconds |
| | hindlimbs | from 5 (inclusive) to 10 (exclusive) seconds; |
| | or forelimbs | less than 5 seconds |
| | hindlimbs | 5 seconds or more |
| 4: | forelimbs | 10 seconds or more |
| | hindlimbs | from 5 (inclusive) to 10 (exclusive) seconds; |
| | or forelimbs | from 5 (inclusive) to 10 (exclusive) seconds |
| | hindlimbs | 10 seconds or more |
| 5: | forelimbs | 10 seconds or more |
| | hindlimbs | 10 seconds or more |

The effect of the compounds was evaluated by the total of the catalepsy scores of five mice in each group (25 points at the full). The groups wherein the total score was not more than 20 points were estimated to be effective. The number of the animals showing remission against catalepsy is the number of the mice for which the catalepsy score was not more than 4 points. The remission rate shows the rate of decrease in total score based on that of the control group.

The $ED_{50}$ (50% effective dose) values were determined using ten mice at each dose. A test compound was judged to be effective at the dose where the catalepsy score was 3 or less than 3. The $ED_{50}$ values were calculated by Probit analysis.

The results are shown in Table 6.

Table 6-1

| Compound | Total Score | Number of the Animals Showing Remission | Remission Rate (%) | $ED_{50}$ (mg/kg) |
|---|---|---|---|---|
| 0.3% Tween 80 (Control) | 25 | 0 | 0 | |
| L-DOPA + benserazide | 18 | 4 | 28 | 107.5 |
| 2 | 2 | 5 | 80 | 0.03 |
| 6 | 6 | 5 | 76 | 1.7 |
| 8 | 2 | 5 | 92 | 0.23 |
| 10 | 8 | 5 | 68 | 0.24 |
| 12 | 12 | 5 | 52 | 2.7 |
| 14 | 1 | 5 | 92 | 0.6 |
| 16 | 4 | 5 | 84 | 0.76 |
| 18 | 4 | 5 | 84 | 1.9 |
| 20 | 7 | 5 | 72 | 0.35 |
| 21 | 19 | 4 | 24 | |
| 22 | 20 | 3 | 20 | |
| 31 | 16 | 4 | 36 | |
| 32 | 17 | 4 | 32 | |
| 34 | 8 | 5 | 68 | 2.6 |

Table 6-1 (continued)

| Compound | Total Score | Number of the Animals Showing Remission | Remission Rate (%) | $ED_{50}$ (mg/kg) |
|---|---|---|---|---|
| 0.3% Tween 80 (Control) | 25 | 0 | 0 | |
| 36 | 6 | 5 | 76 | 1.5 |
| 38 | 8 | 4 | 68 | 2.5 |
| 40 | 3 | 5 | 88 | |
| 44 | 17 | 3 | 32 | |

Table 6-2

| Compound | Total Score | Number of the Animals Showing Remission | Remission Rate (%) | $ED_{50}$ (mg/kg) |
|---|---|---|---|---|
| 0.3% Tween 80 (Control) | 25 | 0 | 0 | |
| L-DOPA + benserazide | 18 | 4 | 28 | 107.5 |
| 67 | 9 | 5 | 64 | |
| 68 | 5 | 5 | 80 | |
| 82 | 9 | 5 | 64 | |

Experimental Example 5 Augmentation of the Contralateral Rotation in Rats with a 6-Hydroxydopamine-Induced Unilateral Lesion of the Nigrostriatal Dopamine Pathway

When a unilateral lesion of the nigrostriatal pathway is induced by 6-hydroxydopamine in rodents, the sensitivity of dopamine receptors in the denervated striatum is enhanced. Administration of a dopamine agonist to the rodents in such a condition induces a rotational behavior to the side contralateral to the lesioned side [Acta Physiol. Scand., 367, 69 (1971)]. This model has been used for a long time as a model for the study of Parkinson's disease and in the screening of drugs for this disease [Neurol. Neurobiol., 33, 1 (1987)].

Male Sprague-Dawley rats (weighing 200 to 240 g, Japan SLC) were pretreated with desipramine hydrochloride (25 mg/kg, i.p., Sigma Co.) 30 minutes before surgery to protect noradrenergic neurons. Then, the animals were anesthetized with sodium pentobarbital (30 mg/kg, i.p., Dainippon Pharm. Co., Ltd.) and the nigrostriatal pathway was lesioned by injection of 6-hydroxydopamine hydrobromide (8 μg, Sigma Co.) into the left medial forebrain bundle. 6-Hydroxydopamine hydrobromide was dissolved in physiological saline containing 0.05% L-ascorbic acid (Wako Pure Chem. Industries, Ltd.) to make 2 μl of solution and injected over 3 minutes.

More than 10 days after surgery, each rat was placed in a plastic bowl (30 cm in diameter). Apomorphine (0.1 mg/kg, Sandoz, AG) was injected subcutaneously and the rats which showed a rotational behavior to the side contralateral to the lesioned side at a frequency of more than 600 counts/60 minutes after apomorphine administration were used for screening. The number of rotations was counted with an automated rotometer, in which each 180° turn was counted as a rotation.

Test compounds were suspended in 0.3% sodium carboxymethylcellulose and administered orally at a dose of 1 mg/kg 30 minutes before the injection of apomorphine (0.1 mg/kg, s.c.). The counts of rotations were summed up every 5 minutes for 150 minutes after apomorphine administration. The total rotation counts induced by apomorphine (0.1 mg/kg, s.c.) with and without a test compound were statistically compared, using the same animals. Rats were allowed to rest more than 5 days between each experiment. Statistical comparison of the values was carried out by Sign-Wilcoxon test.

The results are shown in Table 7.

Table 7

| | total amount of rotations (average count ± S.E.M.) | |
|---|---|---|
| Compd. | apomorphine | test compound + apomorphine |
| 2 | 1102 ± 94 | 1584 ± 196* |

*: $p<0.05$

Table 7 (continued)

| | total amount of rotations (average count ± S.E.M.) | |
|---|---|---|
| Compd. | apomorphine | test compound + apomorphine |
| 8 | 1003 ± 84 | 1406 ± 155* |
| 10 | 1097 ± 147 | 1637 ± 127* |
| 14 | 1006 ± 81 | 1378 ± 216* |

*: $p<0.05$

Compounds (I) and pharmaceutically acceptable salts thereof can be administered as they are, or in the form of various pharmaceutical compositions. The pharmaceutical compositions in accordance with the present invention can be prepared by uniformly mixing an effective amount of Compound (I) or a pharmaceutically acceptable salt thereof, as an active ingredient, with a pharmaceutically acceptable carrier. It is desired that such pharmaceutical compositions are prepared in a unit dose form suitable for oral administration or administration through injection.

For preparing a pharmaceutical composition for oral administration, any useful pharmaceutically acceptable carrier can be used. For example, liquid preparations for oral administration such as suspension and syrup can be prepared using water, sugars such as sucrose, sorbitol and fructose, glycols such as polyethylene glycol and propylene glycol, oils such as sesame oil, olive oil and soybean oil, preservatives such as p-hydroxybenzoates, flavors such as strawberry flavor and peppermint, and the like. Powders, pills, capsules and tablets can be prepared using excipients such as lactose, glucose, sucrose and mannitol, disintegrating agents such as starch and sodium alginate, lubricants such as magnesium stearate and talc, binders such as polyvinyl alcohol, hydroxypropyl cellulose and gelatin, surfactants such as fatty acid esters, plasticizers such as glycerin, and the like. Tablets and capsules are most useful oral unit dose forms because of the readiness of administration. For preparing tablets and capsules, solid pharmaceutical carriers are used.

Solutions as injectable preparations can be prepared using a carrier such as distilled water, a salt solution, a glucose solution or a mixture of a salt solution and a glucose solution. The preparations can be prepared in the form of solution, suspension or dispersion according to a conventional method by using a suitable auxiliary.

Compounds (I) and pharmaceutically acceptable salts thereof can be administered orally in the said dosage forms or parenterally as injections. The effective dose and the administration schedule vary depending upon mode of administration, age, body weight and conditions of a patient, etc. However, generally, Compound (I) or a pharmaceutically acceptable salt thereof is administered in a daily dose of 0.01 to 25 mg/kg in 3 to 4 parts.

In addition, Compounds (I) may also be administered by inhalation in the form of aerosol, fine powder, or spray solution. In the case of aerosol administration, the compound of the present invention is dissolved in an appropriate pharmaceutically acceptable solvent such as ethyl alcohol and a combination of miscible solvents, and the resulting solution is mixed with a pharmaceutically acceptable propellant.

Certain embodiments of the invention are illustrated in the following examples and reference examples.

<u>Example 1</u>

(E)-8-(3,4-Dimethoxystyryl)-1,3-diethylxanthine (Compound 1)

3,4-Dimethoxycinnamic acid (1.39 g, 6.67 mmol) and 3-(3-diethylaminopropyl)-1-ethylcarbodiimide hydrochloride (1.74 g, 9.09 mmol) were added to a mixture of dioxane (40 ml) and water (20 ml) containing 5,6-diamino-1,3-diethyluracil [J. Am. Chem. Soc., 75, 114 (1953)] (1.20 g, 6.06 mmol). The resultant solution was stirred at room temperature for 2 hours at pH 5.5. After neutralization, the reaction solution was extracted three times with 50 ml of chloroform. The combined extract was washed with a saturated aqueous solution of sodium chloride and dried over anhydrous sodium sulfate, followed by evaporation under reduced pressure.

To the residue were added 10 ml of dioxane and 15 ml of an aqueous 1N sodium hydroxide solution, followed by heating under reflux for 20 minutes. After cooling, the solution was neutralized and 20 ml of chloroform was added thereto. The organic layer was separated and the aqueous layer was extracted twice with 20 ml of chloroform. The combined extract was washed with a saturated aqueous solution of sodium chloride and dried over anhydrous sodium sulfate, followed by evaporation under reduced pressure. The residue was purified by silica gel column chromatography (eluent: 2% methanol/chloroform), followed by recrystallization from toluene to give 1.06 g (yield 47%) of Compound 1 as pale yellow needles.

Melting Point: 268.8-269.1°C

| Elemental Analysis: $C_{19}H_{22}N_4O_4$ | | | |
|---|---|---|---|
| Calcd. (%) | C, 61.61; | H, 5.98; | N, 15.12 |
| Found (%) | C, 61.99; | H, 6.00; | N, 14.91 |

IR (KBr) $\nu_{max}$ ($cm^{-1}$): 1694, 1641, 1514, 1492

NMR (270MHz; DMSO-$d_6$) δ (ppm): 13.35(1H, brs), 7.59 (1H, d, J=16.2Hz), 7.27(1H, d, J=1.4Hz), 7.14(1H, dd, J=1.4, 8.2Hz), 6.99(1H, d, J=8.2Hz), 6.96(1H, d, J=16.2Hz), 4.06(2H, q, J=7.0Hz), 3.91(2H, q, J=7.0Hz), 3.83(3H, s), 3.79(3H, s), 1.26(3H, t, J=7.0Hz), 1.14(3H, t, J=7.0Hz)

Example 2

(E)-8-(3,4-Dimethoxystyryl)-1,3-diethyl-7-methyl-xanthine (Compound 2)

Compound 1 (1.20 g, 3.24 mmol) obtained in Example 1 was dissolved in 25 ml of dimethylformamide. To the solution were added 1.12 g (8.10 mmol) of potassium carbonate and subsequently 0.40 ml (6.49 mmol) of methyl iodide, and the resultant mixture was stirred at 50°C for 30 minutes. After cooling, insoluble matters were filtered off, and 100 ml of water was added to the filtrate. The mixture was extracted three times with 50 ml of chloroform. The extract was washed twice with water and once with a saturated aqueous solution of sodium chloride, and dried over anhydrous sodium sulfate, followed by evaporation under reduced pressure. The obtained crude crystals were purified by silica gel column chromatography (eluent: 40% ethyl acetate/hexane), followed by recrystallization from isopropanol to give 840 mg (yield 68%) of Compound 2 as pale yellow needles.

Melting Point: 190.4-191.3°C

| Elemental Analysis: $C_{20}H_{24}N_4O_4$ | | | |
|---|---|---|---|
| Calcd. (%) | C, 62.48; | H, 6.29; | N, 14.57 |
| Found (%) | C, 62.52; | H, 6.53; | N, 14.56 |

IR (KBr) $\nu_{max}$ ($cm^{-1}$): 1697, 1655, 1518

NMR (270MHz; $CDCl_3$) δ (ppm): 7.74(1H, d, J=15.5Hz), 7.18(1H, dd, J=1.9, 8.3Hz), 7.08(1H, d, J=1.9Hz), 6.89 (1H, d, J=8.3Hz), 6.77(1H, d, J=15.5Hz), 4.21 (2H, q, J=6.9Hz), 4.09(2H, q, J=6.9Hz), 4.06(3H, s), 3.96(3H, s), 3.93 (3H, s), 1.39(3H, t, J=6.9Hz), 1.27(3H, t, J=6.9Hz)

Example 3

(E)-8-(2,3-Dimethoxystyryl)-1,3-diethylxanthine (Compound 3)

Substantially the same procedure as in Example 1 was repeated using 2.0 g (10.1 mmol) of 5,6-diamino-1,3-diethyluracil and 2.52 g (12.1 mmol) of 2,3-dimethoxycinnamic acid. Then, the resultant crude crystals were recrystallized from dimethylsulfoxide/water to give 1.72 g (yield 46%) of Compound 3 as a white powder.

Melting Point: 287.5-289.4°C

| Elemental Analysis: $C_{19}H_{22}N_4O_4$ | | | |
|---|---|---|---|
| Calcd. (%) | C, 61.61; | H, 5.98; | N, 15.12 |
| Found (%) | C, 61.56; | H, 6.11; | N, 14.83 |

IR (KBr) $\nu_{max}$ ($cm^{-1}$): 1697, 1656, 1500

NMR (270MHz; DMSO-$d_6$) δ (ppm): 13.64(1H, brs), 7.84 (1H, d, J=16.8Hz), 7.29(1H, dd, J=1.7, 7.6Hz), 7.15-7.00 (3H, m), 4.07(2H, q, J=7.0Hz), 3.94(2H, q, J=7.0Hz), 3.83(3H, s), 3.79(3H, s), 1.26(3H, t, J=7.0Hz), 1.14(3H, t, J=7.0Hz)

Example 4

(E)-8-(2,3-Dimethoxystyryl)-1,3-diethyl-7-methyl-xanthine (Compound 4)

Substantially the same procedure as in Example 2 was repeated using 1.60 g (4.32 mmol) of Compound 3

obtained in Example 3 in place of Compound 1. Then, the resultant crude crystals were recrystallized from cyclohexane/toluene to give 1.21 g (yield 73%) of Compound 4 as a pale yellow powder.

Melting Point: 194.9-195.6°C

| Elemental Analysis: $C_{20}H_{24}N_4O_4$ | | | |
|---|---|---|---|
| Calcd. (%) | C, 62.48; | H, 6.29; | N, 14.57 |
| Found (%) | C, 62.67; | H, 6.48; | N, 14.31 |

IR (KBr) $\nu_{max}$ ($cm^{-1}$): 1694, 1660, 1272

NMR (270MHz; $CDCl_3$) δ (ppm): 8.00(1H, d, J=16.8Hz), 7.19(1H, dd, J=1.3, 7.9Hz), 7.15-7.00(2H, m), 6.93 (1H, dd, J=1.3, 7.9Hz), 4.26(2H, q, J=6.9Hz), 4.09 (2H, q, J=6.9Hz), 4.05(3H, s), 3.91(3H, s), 3.90 (3H, s), 1.39(3H, t, J=6.9Hz), 1.27(3H, t, J=6.9Hz)

Example 5

(E)-8-(2,4-Dimethoxystyryl)-1,3-diethylxanthine (Compound 5)

Substantially the same procedure as in Example 1 was repeated using 2.50 g (12.6 mmol) of 5,6-diamino-1,3-diethyluracil and 2.89 g (13.9 mmol) of 2,4-dimethoxycinnamic acid. Then, the resultant crude crystals were recrystallized from dimethylformamide/ethanol to give 0.92 g (yield 20%) of Compound 5 as yellow crystals.

Melting Point: 278.7-279.8°C

| Elemental Analysis: $C_{19}H_{22}N_4O_4$ | | | |
|---|---|---|---|
| Calcd. (%) | C, 61.61; | H, 5.98; | N, 15.12 |
| Found (%) | C, 61.65; | H, 5.95; | N, 14.74 |

IR (KBr) $\nu_{max}$ ($cm^{-1}$): 1698, 1640, 1509, 1292

NMR (270MHz; DMSO-$d_6$) δ (ppm): 13.43(1H, brs), 7.77 (1H, d, J=16.8Hz), 7.54(1H, d, J=8.4Hz), 6.95(1H, d, J=16.8Hz), 6.63(1H, d, J=2.5Hz), 6.60(1H, dd, J=2.5, 8.4Hz), 4.06(2H, q, J=6.9Hz), 3.93(2H, q, J=6.9Hz), 3.89(3H, s), 3.82(3H, s), 1.25(3H, t, J=6.9Hz), 1.13(3H, t, J=6.9Hz)

Example 6

(E)-8-(2,4-Dimethoxystyryl)-1,3-diethyl-7-methylxanthine (Compound 6)

Substantially the same procedure as in Example 2 was repeated using 400 mg (1.08 mmol) of Compound 5 obtained in Example 5 in place of Compound 1. Then, the resultant crude crystals were recrystallized from hexane/ethyl acetate to give 335 mg (yield 81%) of Compound 6 as yellow needles.

Melting Point: 195.9-196.7°C

| Elemental Analysis: $C_{20}H_{24}N_4O_4$ | | | |
|---|---|---|---|
| Calcd. (%) | C, 62.48; | H, 6.29; | N, 14.57 |
| Found (%) | C, 62.29; | H, 6.51; | N, 14.66 |

IR (KBr) $\nu_{max}$ ($cm^{-1}$): 1693, 1654, 1603, 1294

NMR (270MHz; $CDCl_3$) δ (ppm): 7.93(1H, d, J=15.8Hz), 7.48(1H, d, J=8.3Hz), 6.97(1H, d, J=15.8Hz), 6.53 (1H, dd, J=2.0, 8.3Hz), 6.49(1H, d, J=2.0Hz), 4.22 (2H, q, J=6.9Hz), 4.08(2H, q, J=6.9Hz), 4.02(3H, s), 3.92(3H, s), 3.86 (3H, s), 1.38(3H, t, J=6.9Hz), 1.26(3H, t, J=6.9Hz)

Example 7

(E)-1,3-Diethyl-8-(2,3,4-trimethoxystyryl)xanthine (Compound 7)

Substantially the same procedure as in Example 1 was repeated using 2.5 g (12.6 mmol) of 5,6-diamino-1,3-diethyluracil and 2.3 g (13.9 mmol) of 2,3,4-trimethoxycinnamic acid. Then, the resultant crude crystals were recrystallized from dioxane/water to give 2.85 g (yield 57%) of Compound 7 as white crystals.

Melting Point: 276.3-277.0°C

| Elemental Analysis: $C_{20}H_{24}N_4O_5$ | | | |
|---|---|---|---|
| Calcd. (%) | C, 59.99; | H, 6.04; | N, 13.99 |
| Found (%) | C, 60.26; | H, 6.24; | N, 14.28 |

IR (KBr) $\nu_{max}$ ($cm^{-1}$): 1696, 1655, 1500

NMR (270MHz; $CDCl_3$) δ (ppm): 12.39(1H, brs), 7.88(1H, d, J=16.3Hz), 7.30(1H, d, J=8.4Hz), 7.09(1H, d, J=16.3Hz), 6.73(1H, d, J=8.4Hz), 4.26(2H, q, J=6.9Hz), 4.20(2H, q, J=6.9Hz), 3.96(3H, s), 3.92 (3H, s), 3.91(3H, s), 1.41(3H, t, J=6.9Hz), 1.29 (3H, t, J=6.9Hz)

Example 8

(E)-1,3-Diethyl-7-methyl-8-(2,3,4-trimethoxystyryl)-xanthine (Compound 8)

Substantially the same procedure as in Example 2 was repeated using 1.5 g (3.75 mmol) of Compound 7 obtained in Example 7 in place of Compound 1. Then, the resultant crude crystals were recrystallized from hexane/ethyl acetate to give 1.32 g (yield 85%) of Compound 8 as colorless needles.

Melting Point: 152.9-154.3°C

| Elemental Analysis: $C_{21}H_{26}N_4O_5$ | | | |
|---|---|---|---|
| Calcd. (%) | C, 60.86; | H, 6.32; | N, 13.52 |
| Found (%) | C, 61.04; | H, 6.44; | N, 13.79 |

IR (KBr) $\nu_{max}$ ($cm^{-1}$): 1695, 1655, 1498, 1289

NMR (270MHz; $CDCl_3$) δ (ppm): 7.88(1H, d, J=15.8Hz), 7.28(1H, d, J=8.9Hz), 7.01(1H, d, J=15.8Hz), 6.72 (1H, d, J=8.9Hz), 4.22(2H, q, J=6.9Hz), 4.09(2H, q, J=6.9Hz), 4.04(3H, s), 3.97(3H, s), 3.91(3H, s), 3.90(3H, s), 1.38(3H, t, J=6.9Hz), 1.27(3H, t, J=6.9Hz)

Example 9

(E)-1,3-Diethyl-8-(4-methoxy-2,3-dimethylstyryl)-xanthine (Compound 9)

Substantially the same procedure as in Example 1 was repeated using 2.5 g (12.6 mmol) of 5,6-diamino-1,3-diethyluracil and 2.9 g (13.9 mmol) of 4-methoxy-2,3-dimethylcinnamic acid. Then, the resultant crude crystals were recrystallized from ethanol/water to give 0.80 g (yield 17%) of Compound 9 as white crystals.

Melting Point: >280.0°C

| Elemental Analysis: $C_{20}H_{24}N_4O_3$ | | | |
|---|---|---|---|
| Calcd. (%) | C, 65.20; | H, 6.56; | N, 15.21 |
| Found (%) | C, 65.24; | H, 6.61; | N, 15.29 |

IR (KBr) $\nu_{max}$ ($cm^{-1}$): 1697, 1642, 1496, 1270

NMR (270MHz; DMSO-$d_6$) 6 (ppm): 13.52(1H, brs), 7.93 (1H, d, J=15.8Hz), 7.56(1H, d, J=8.2Hz), 6.89(1H, d, J=8.2Hz), 6.82(1H, d, J=15.8Hz), 4.06(2H, q, J=6.9Hz), 3.94(2H, q, J=6.9Hz), 3.81(3H, s), 2.33 (3H, s), 2.13(3H, s), 1.26(3H, t, J=6.9Hz), 1.14 (3H, t, J=6.9Hz)

Example 10

(E)-1,3-Diethyl-8-(4-methoxy-2,3-dimethylstyryl)-7-methylxanthine (Compound 10)

Substantially the same procedure as in Example 2 was repeated using 500 mg (1.36 mmol) of Compound 9 obtained in Example 9 in place of Compound 1. Then, the resultant crude crystals were recrystallized from hexane/ethyl acetate to give 493 mg (yield 95%) of Compound 10 as pale yellow needles.

Melting Point: 207.7-208.3°C

| Elemental Analysis: $C_{21}H_{26}N_4O_3$ | | | |
|---|---|---|---|
| Calcd. (%) | C, 65.95; | H, 6.85; | N, 14.65 |
| Found (%) | C, 66.24; | H, 6.99; | N, 14.69 |

IR (KBr) $\nu_{max}$ ($cm^{-1}$): 1698, 1651, 1267

NMR (270MHz; $CDCl_3$) δ (ppm): 8.08(1H, d, J=15.2Hz), 7.46(1H, d, J=8.9Hz), 6.77(1H, d, J=8.9Hz), 6.67 (1H, d, J=15.2Hz), 4.22(2H, q, J=6.9Hz), 4.09(2H, q, J=6.9Hz), 4.03(3H, s), 3.86(3H, s), 2.40(3H, s), 2.21(3H, s), 1.39(3H, t, J=6.9Hz), 1.26(3H, t, J=6.9Hz)

Example 11

(E)-1,3-Diethyl-8-(4-methoxy-2,5-dimethylstyryl)-xanthine (Compound 11)

Substantially the same procedure as in Example 1 was repeated using 2.5 g (12.6 mmol) of 5,6-diamino-1,3-diethyluracil and 2.9 g (13.9 mmol) of 4-methoxy-2,5-dimethylcinnamic acid. Then, the resultant crude crystals were recrystallized from dioxane/water to give 2.43 g (yield 52%) of Compound 11 as white crystals.

Melting Point: >280.0°C

| Elemental Analysis: $C_{20}H_{24}N_4O_3$ | | | |
|---|---|---|---|
| Calcd. (%) | C, 65.20; | H, 6.56; | N, 15.21 |
| Found (%) | C, 64.83; | H, 6.56; | N, 15.43 |

IR (KBr) $\nu_{max}$ ($cm^{-1}$): 1690, 1646, 1510, 1265

NMR (270MHz; DMSO-$d_6$) δ (ppm): 13.52(1H, brs), 7.82 (1H, d, J=16.3Hz), 7.54(1H, s), 6.86(1H, d, J=16.3Hz), 6.82(1H, s), 4.06(2H, q, J=6.9Hz), 3.94 (2H, q, J=6.9Hz), 3.81(3H, s), 2.41(3H, s), 2.14 (3H, s), 1.25(3H, t, J=6.9Hz), 1.14(3H, t, J=6.9Hz)

Example 12

(E)-1,3-Diethyl-8-(4-methoxy-2,5-dimethylstyryl)-7-methylxanthine (Compound 12)

Substantially the same procedure as in Example 2 was repeated using 1.10 g (2.98 mmol) of Compound 11 obtained in Example 11 in place of Compound 1. Then, the resultant crude crystals were recrystallized from ethyl acetate to give 0.76 g (yield 67%) of Compound 12 as yellow needles.

Melting Point: 235.4-236.1°C

| Elemental Analysis: $C_{21}H_{26}N_4O_3$ | | | |
|---|---|---|---|
| Calcd. (%) | C, 65.95; | H, 6.85; | N, 14.65 |
| Found (%) | C, 65.56; | H, 6.93; | N, 14.64 |

IR (KBr) $\nu_{max}$ ($cm^{-1}$): 1689, 1657, 1510, 1263

NMR (270MHz; $CDCl_3$) δ (ppm): 7.97(1H, d, J=15.5Hz), 7.42(1H, s), 6.71(1H, d, J=15.5Hz), 6.66(1H, s), 4.22 (2H, q, J=6.9Hz), 4.09(2H, q, J=6.9Hz), 4.05 (3H, s), 3.86(3H, s), 2.48(3H, s), 2.23(3H, s), 1.38(3H, t, J=6.9Hz), 1.26 (3H, t, J=6.9Hz)

Example 13

(E)-8-(2,4-Dimethoxy-3-methylstyryl)-1,3-diethyl-xanthine (Compound 13)

Substantially the same procedure as in Example 1 was repeated using 2.0 g (10.1 mmol) of 5,6-diamino-1,3-diethyluracil and 2.04 g (9.19 mmol) of 2,4-dimethoxy-3-methylcinnamic acid. Then, the resultant crude crystals were recrystallized from dioxane/water to give 1.22 g (yield 32%) of Compound 13 as a yellow powder.

Melting Point: >275.0°C

| Elemental Analysis: $C_{20}H_{24}N_4O_4$ | | | |
|---|---|---|---|
| Calcd. (%) | C, 62.48; | H, 6.29; | N, 14.57 |
| Found (%) | C, 62.28; | H, 6.42; | N, 14.22 |

IR (KBr) $\nu_{max}$ ($cm^{-1}$): 1696, 1635, 1592, 1499

NMR (270MHz; DMSO-$d_6$) δ (ppm): 7.75(1H, d, J=16.5Hz), 7.58(1H, d, J=8.8Hz), 6.99(1H, d, J=16.5Hz), 6.85 (1H, d, J=8.8Hz), 4.04(2H, q, J=6.9Hz), 3.95(2H, q, J=6.9Hz), 3.83(3H, s), 3.70(3H, s), 2.09(3H, s), 1.26(3H, t, J=6.9Hz), 1.14(3H, t, J=6.9Hz)

Example 14

(E)-8-(2,4-Dimethoxy-3-methylstyryl)-1,3-diethyl-7-methylxanthine (Compound 14)

Substantially the same procedure as in Example 2 was repeated using 700 mg (1.82 mmol) of Compound 13 obtained in Example 13 in place of Compound 1. Then, the resultant crude crystals were recrystallized from cyclohexane/toluene to give 610 mg (yield 84%) of Compound 14 as pale yellow needles.

Melting Point: 196.1-196.8°C

| Elemental Analysis: $C_{21}H_{26}N_4O_4$ | | | |
|---|---|---|---|
| Calcd. (%) | C, 63.30; | H, 6.57; | N, 14.06 |
| Found (%) | C, 63.32; | H, 6.74; | N, 14.13 |

IR (KBr) $\nu_{max}$ ($cm^{-1}$): 1695, 1649, 1498

NMR (270MHz; $CDCl_3$) δ (ppm): 7.81(1H, d, J=15.8Hz), 7.78(1H, d, J=8.6Hz), 7.23(1H, d, J=15.8Hz), 6.87 (1H, d, J=8.6Hz), 4.07(2H, q, J=6.9Hz), 4.01(3H, s), 3.92(2H, q, J=6.9Hz), 3.85(3H, s), 3.70(3H, s), 2.10(3H, s), 1.27(3H, t, J=6.9Hz), 1.13(3H, t, J=6.9Hz)

Example 15

(E)-1,3-Diethyl-8-(3,4-methylenedioxystyryl)xanthine (Compound 15)

Substantially the same procedure as in Example 1 was repeated using 2.0 g (10.1 mmol) of 5,6-diamino-1,3-diethyluracil and 2.33 g (12.1 mmol) of 3,4-methylenedioxycinnamic acid. Then, the resultant crude crystals were recrystallized from dimethylformamide/water to give 1.34 g (yield 38%) of Compound 15 as a yellowish green powder.

Melting Point: >275.0°C

| Elemental Analysis: $C_{18}H_{18}N_4O_4$ | | | |
|---|---|---|---|
| Calcd. (%) | C, 61.01; | H, 5.11; | N, 15.81 |
| Found (%) | C, 61.16; | H, 5.03; | N, 15.80 |

IR (KBr) $\nu_{max}$ ($cm^{-1}$): 1685, 1638, 1499

NMR (270MHz; DMSO-$d_6$) δ (ppm): 7.55(1H, d, J=16.3Hz), 7.30(1H, s), 7.08(1H, d, J=8.9Hz), 6.96(1H, d, J=8.9Hz), 6.90(1H, d, J=16.3Hz), 6.07(2H, s), 4.05 (2H, q, J=6.9Hz), 3.93(2H, q, J=6.9Hz), 1.25(3H, t, J=6.9Hz), 1.10 (3H, t, J=6.9Hz)

Example 16

(E)-1,3-Diethyl-7-methyl-8-(3,4-methylenedioxystyryl)-xanthine (Compound 16)

Substantially the same procedure as in Example 2 was repeated using 1.35 g (3.81 mmol) of Compound 15 obtained in Example 15 in place of Compound 1. Then, the resultant crude crystals were recrystallized from cyclohexane/toluene to give 940 mg (yield 67%) of Compound 16 as yellow needles.

Melting Point: 219.4-219.6°C

| Elemental Analysis: $C_{19}H_{20}N_4O_4$ | | | |
|---|---|---|---|
| Calcd. (%) | C, 61.94; | H, 5.47; | N, 15.20 |
| Found (%) | C, 62.09; | H, 5.41; | N, 15.16 |

IR (KBr) $\nu_{max}$ ($cm^{-1}$): 1687, 1657, 1569, 1498, 1443

NMR (270MHz; $CDCl_3$) δ (ppm): 7.70(1H, d, J=15.5Hz), 7.10(1H, d, J=1.6Hz), 7.06(1H, dd, J=1.6, 8.0Hz), 6.84 (1H, d, J=8.0Hz), 6.73(1H, d, J=15.5Hz), 6.02 (2H, s), 4.21(2H, q, J=6.9Hz), 4.09(2H, q, J=6.9Hz), 4.04(3H, s), 1.38 (3H, t, J=6.9Hz), 1.26 (3H, t, J=6.9Hz)

Example 17

(E)-8-[2-(1,4-Benzodioxan-6-yl)vinyl]-1,3-diethyl-xanthine (Compound 17)

Substantially the same procedure as in Example 1 was repeated using 2.85 g (14.4 mmol) of 5,6-diamino-1,3-diethyluracil and 2.70 g (13.1 mmol) of 3-(1,4-benzodioxan-6-yl)acrylic acid. Then, the resultant crude crystals were recrystallized from dioxane/water to give 2.45 g (yield 51%) of Compound 17 as a pale yellow powder.

Melting Point: >300°C

| Elemental Analysis: $C_{19}H_{20}N_4O_4$ | | | |
|---|---|---|---|
| Calcd. (%) | C, 61.94; | H, 5.47; | N, 15.20 |
| Found (%) | C, 61.97; | H, 5.62; | N, 15.07 |

IR (KBr) $\nu_{max}$ ($cm^{-1}$): 1682, 1637, 1511, 1310

NMR (270MHz; DMSO-$d_6$) δ (ppm): 7.51(1H, d, J=16.2Hz), 7.10-7.03(2H, m), 6.89(1H, d, J=7.9Hz), 6.87(1H, d, J=16.2Hz), 4.27(4H, s), 4.05(2H, q, J=6.9Hz), 3.93(2H, q, J=6.9Hz), 1.22(3H, t, J=6.9Hz), 1.13 (3H, t, J=6.9Hz)

Example 18

(E)-8-[2-(1,4-Benzodioxan-6-yl)vinyl]-1,3-diethyl-7-methylxanthine (Compound 18)

Substantially the same procedure as in Example 2 was repeated using 2.00 g (5.43 mmol) of Compound 17 obtained in Example 17 in place of Compound 1. Then, the resultant crude crystals were recrystallized from ethanol/isopropanol to give 1.58 g (yield 76%) of Compound 18 as yellow needles.

Melting Point: 233.1-233.6°C

| Elemental Analysis: $C_{20}H_{22}N_4O_4$ | | | |
|---|---|---|---|
| Calcd. (%) | C, 62.81; | H, 5.79; | N, 14.65 |
| Found (%) | C, 62.55; | H, 5.80; | N, 14.60 |

IR (KBr) $\nu_{max}$ ($cm^{-1}$): 1689, 1654, 1509

NMR (270MHz; $CDCl_3$) δ (ppm): 7.67(1H, d, J=15.8Hz), 7.15-7.05(2H, m), 6.88(1H, d, J=8.3Hz), 6.75(1H, d, J=15.8Hz), 4.30(4H, s), 4.21(2H, q, J=6.9Hz), 4.08(2H, q, J=6.9Hz), 4.03(3H, s), 1.39(3H, t, J=6.9Hz), 1.35(3H, t, J=6.9Hz)

Example 19

(E)-8-(2,3,4-Trimethoxystyryl)theophylline (Compound 19)

Substantially the same procedure as in Example 1 was repeated using 5.00 g (29.4 mmol) of 5,6-diamino-1,3-dimethyluracil and 7.71 g (32.4 mmol) of 2,3,4-trimethoxycinnamic acid. Then, the resultant crude crystals were recrystallized from isopropanol/water to give 3.78 g (yield 35%) of Compound 19 as an ocher powder.

Melting Point: 264.8-266.1°C

| Elemental Analysis: $C_{18}H_{20}N_4O_5$ | | | |
|---|---|---|---|
| Calcd. (%) | C, 58.05; | H, 5.41; | N, 15.04 |
| Found (%) | C, 58.28; | H, 5.38; | N, 15.20 |

IR (KBr) $\nu_{max}$ ($cm^{-1}$): 1697, 1651, 1505, 1297

NMR (270MHz; $CDCl_3$) δ (ppm): 12.78(1H, s), 7.91(1H, d, J=16.8Hz), 7.28(1H, d, J=9.4Hz), 7.13(1H, d, J=16.8Hz), 6.73(1H, d, J=9.4Hz), 3.95(3H, s), 3.92 (3H, s), 3.90(3H, s), 3.69(3H, s), 3.54(3H, s)

Example 20

(E)-8-(2,3,4-Trimethoxystyryl)caffeine (Compound 20)

Substantially the same procedure as in Example 2 was repeated using 2.00 g (5.38 mmol) of Compound 19 obtained in Example 19 in place of Compound 1. Then, the resultant crude crystals were recrystallized from cyclohexane/toluene to give 1.68 g (yield 81%) of Compound 20 as a pale yellow powder.

Melting Point: 186.7-187.9°C

| Elemental Analysis: $C_{19}H_{22}N_4O_5$ | | | |
|---|---|---|---|
| Calcd. (%) | C, 59.06; | H, 5.74; | N, 14.50 |
| Found (%) | C, 59.27; | H, 5.72; | N, 14.60 |

IR (KBr) $\nu_{max}$ ($cm^{-1}$): 1694, 1655, 1596, 1544, 1501, 1295

NMR (270MHz; $CDCl_3$) δ (ppm): 7.90(1H, d, J=16.3Hz), 7.28(1H, d, J=7.9Hz), 7.01(1H, d, J=16.3Hz), 6.72 (1H, d, J=7.9Hz), 4.04(3H, s), 3.97(3H, s), 3.91 (3H, s), 3.90(3H, s), 3.64(3H, s), 3.42(3H, s)

Example 21

(E)-8-(4-Methoxy-2,3-dimethylstyryl)theophylline (Compound 21)

Substantially the same procedure as in Example 1 was repeated using 1.74 g (10.2 mmol) of 5,6-diamino-1,3-dimethyluracil and 2.42 g (11.8 mmol) of 4-methoxy-2,3-dimethylcinnamic acid. Then, the resultant crude crystals were recrystallized from acetonitrile to give 750 mg (yield 22%) of Compound 21 as a white powder.

Melting Point: >275°C

| Elemental Analysis: $C_{18}H_{20}N_4O_3$ | | | |
|---|---|---|---|
| Calcd. (%) | C, 63.51; | H, 5.92; | N, 16.46 |
| Found (%) | C, 63.56; | H, 5.82; | N, 16.30 |

IR (KBr) $\nu_{max}$ ($cm^{-1}$): 1703, 1634, 1593

NMR (270MHz; DMSO-$d_6$) δ (ppm): 13.45(1H, s), 7.93(1H, d, J=16.2Hz), 7.53(1H, d, J=8.9Hz), 6.88(1H, d, J=8.9Hz), 6.79(1H, d, J=16.2Hz), 3.80(3H, s), 3.75 (3H, s), 3.25(3H, s), 2.32(3H, s), 2.12(3H, s)

Example 22

(E)-8-(4-Methoxy-2,3-dimethylstyryl)caffeine (Compound 22)

Substantially the same procedure as in Example 2 was repeated using 500 mg (1.47 mmol) of Compound 21 obtained in Example 21 in place of Compound 1. Then, the resultant crude crystals were recrystallized from toluene to give 280 mg (yield 54%) of Compound 22 as a pale yellow powder.

Melting Point: >275°C

| Elemental Analysis: $C_{19}H_{22}N_4O_3$ | | | |
|---|---|---|---|
| Calcd. (%) | C, 64.39; | H, 6.25; | N, 15.80 |

(continued)

| Elemental Analysis: $C_{19}H_{22}N_4O_3$ | | | |
|---|---|---|---|
| Found (%) | C, 64.44; | H, 6.27; | N, 16.11 |

IR (KBr) $\nu_{max}$ ($cm^{-1}$): 1694, 1650, 1544, 1491, 1435

NMR (270MHz; $CDCl_3$) δ (ppm): 7.96(1H, d, J=15.5Hz), 7.73(1H, d, J=8.6Hz), 7.07(1H, d, J=15.5Hz), 6.90 (1H, d, J=8.6Hz), 4.02(3H, s), 3.82(3H, s), 3.48 (3H, s), 3.29(3H, s), 2.32(3H, s), 2.13(3H, s)

Example 23

(E)-8-(3,4-Methylenedioxystyryl)theophylline (Compound 23)

Substantially the same procedure as in Example 1 was repeated using 5.0 g (29.4 mmol) of 5,6-diamino-1,3-dimethyluracil and 6.78 g (35.3 mmol) of 3,4-methylenedioxycinnamic acid. Then, the resultant crude crystals were recrystallized from dimethylformamide/water to give 1.20 g (yield 13%) of Compound 23 as a pale yellow powder.

Melting Point: >275°C

| Elemental Analysis: $C_{16}H_{14}N_4O_4$ | | | |
|---|---|---|---|
| Calcd. (%) | C, 58.99; | H, 4.32; | N, 17.16 |
| Found (%) | C, 58.84; | H, 4.30; | N, 16.97 |

IR (KBr) $\nu_{max}$ ($cm^{-1}$): 1692, 1642, 1499

NMR (270MHz; DMSO-$d_6$) δ (ppm): 7.57(1H, d, J=16.1Hz), 7.09(1H, s), 7.07(1H, d, J=7.9Hz), 6.92(1H, d, J=7.9Hz), 6.88(1H, d, J=16.lHz), 6.07(2H, s), 3.47 (3H, s), 3.30(3H, s)

Example 24

(E)-8-(3,4-Methylenedioxystyryl)caffeine (Compound 24)

Substantially the same procedure as in Example 2 was repeated using 2.32 g (7.13 mmol) of Compound 23 obtained in Example 23 in place of Compound 1. Then, the resultant crude crystals were recrystallized from dioxane to give 1.54 g (yield 64%) of Compound 24 as yellow needles.

Melting Point: >300°C

| Elemental Analysis: $C_{17}H_{16}N_4O_4$ | | | |
|---|---|---|---|
| Calcd. (%) | C, 59.99; | H, 4.73; | N, 16.46 |
| Found (%) | C, 59.98; | H, 4.66; | N, 16.38 |

IR (KBr) $\nu_{max}$ ($cm^{-1}$): 1702, 1663, 1545, 1506

NMR (270MHz; $CDCl_3$) δ (ppm): 7.72(1H, d, J=15.3Hz), 7.10(1H, d, J=1.5Hz), 7.06(1H, dd, J=1.5, 7.9Hz), 6.84 (1H, d, J=7.9Hz), 6.73(1H, d, J=15.3Hz), 6.03 (2H, s), 4.05(3H, s), 3.63(3H, s), 3.42(3H, s)

Example 25

(E)-8-(2,3-Dimethoxystyryl)theophylline (Compound 25)

Substantially the same procedure as in Example 1 was repeated using 2.50 g (14.7 mmol) of 5,6-diamino-1,3-dimethyluracil and 3.37 g (16.2 mmol) of 2,3-dimethoxycinnamic acid. Then, the resultant crude crystals were recrystallized from ethanol/water to give 1.03 g (yield 41%) of Compound 25 as pale yellow needles.

Melting Point: 289.2-290.5°C

| Elemental Analysis: $C_{17}H_{18}N_4O_4$ | | | |
|---|---|---|---|
| Calcd. (%) | C, 59.64; | H, 5.29; | N, 16.36 |
| Found (%) | C, 59.42; | H, 5.12; | N, 16.65 |

IR (KBr) $\nu_{max}$ ($cm^{-1}$): 1700, 1649, 1499, 1476, 1273

NMR (270MHz; DMSO-$d_6$) δ (ppm): 13.60(1H, brs), 7.84 (1H, d, J=16.8Hz), 7.26(1H, d, J=6.9Hz), 7.15-7.00 (3H, m), 3.83(3H, s), 3.79(3H, s), 3.48(3H, s), 3.26(3H, s)

Example 26

(E)-8-(2,3-Dimethoxystyryl)caffeine (Compound 26)

Substantially the same procedure as in Example 2 was repeated using 1.10 g (3.22 mmol) of Compound 25 obtained in Example 25 in place of Compound 1. Then, the resultant crude crystals were recrystallized from toluene to give 570 mg (yield 50%) of Compound 26 as yellow needles.

Melting Point: 233.6-236.7°C

| Elemental Analysis: $C_{18}H_{20}N_4O_4$ | | | |
|---|---|---|---|
| Calcd. (%) | C, 60.66; | H, 5.65; | N, 15.72 |
| Found (%) | C, 60.21; | H, 5.74; | N, 16.13 |

IR (KBr) $\nu_{max}$ ($cm^{-1}$): 1688, 1645, 1545, 1480

NMR (270MHz; DMSO-$d_6$) δ (ppm): 7.91(1H, d, J=16.0Hz), 7.52(1H, dd, J=1.7, 7.6Hz), 7.32(1H, d, J=16.0Hz), 7.10-7.05(2H, m), 4.03(3H, s), 3.84(3H, s), 3.79 (3H, s), 3.48(3H, s), 3.24(3H, s)

Example 27

(E)-8-(2,4-Dimethoxystyryl)theophylline (Compound 27)

Substantially the same procedure as in Example 1 was repeated using 1.0 g (5.88 mmol) of 5,6-diamino-1,3-dimethyluracil and 1.35 g (6.48 mmol) of 2,4-dimethoxycinnamic acid. Then, the resultant crude crystals were recrystallized from dimethylformamide to give 221 mg (yield 11%) of Compound 27 as pale yellow grains.

Melting Point: >280°C

| Elemental Analysis: $C_{17}H_{18}N_4O_4$ | | | |
|---|---|---|---|
| Calcd. (%) | C, 59.64; | H, 5.29; | N, 16.36 |
| Found (%) | C, 59.51; | H, 5.34; | N, 16.58 |

IR (KBr) $\nu_{max}$ ($cm^{-1}$): 1705, 1650, 1607, 1505

NMR (270MHz; DMSO-$d_6$) δ (ppm): 13.40(1H, brs), 7.78 (1H, d, J=16.5Hz), 7.53(1H, d, J=8.3Hz), 6.93(1H, d, J=16.5Hz), 6.63(1H, d, J=2.3Hz), 6.60(1H, dd, J=2.3, 8.3Hz), 3.89(3H, s), 3.82(3H, s), 3.47(3H, s), 3.25(3H, s)

Example 28

(E)-8-(2,4-Dimethoxystyryl)caffeine (Compound 28)

Substantially the same procedure as in Example 2 was repeated using 700 mg (2.05 mmol) of Compound 27 obtained in Example 27 in place of Compound 1. Then, the resultant crude crystals were recrystallized from dioxane to give 621 mg (yield 85%) of Compound 28 as yellow needles.

Melting Point: 241.5-242.1°C

| Elemental Analysis: $C_{18}H_{20}N_4O_4$ | | | |
|---|---|---|---|
| Calcd. (%) | C, 60.66; | H, 5.65; | N, 15.72 |
| Found (%) | C, 60.49; | H, 5.61; | N, 15.69 |

IR (KBr) $\nu_{max}$ ($cm^{-1}$): 1685, 1650, 1602, 1434

NMR (270MHz; $CDCl_3$) δ (ppm): 7.95(1H, d, J=15.8Hz), 7.48(1H, d, J=8.6Hz), 6.98(1H, d, J=15.8Hz), 6.54 (1H, dd, J=2.3, 8.6Hz), 6.49(1H, d, J=2.3Hz), 4.03 (3H, s), 3.92(3H, s), 3.86(3H, s), 3.64(3H, s), 3.42(3H, s)

Example 29

(E)-8-(4-Methoxy-2,5-dimethylstyryl)theophylline (Compound 29)

Substantially the same procedure as in Example 1 was repeated using 1.0 g (5.88 mmol) of 5,6-diamino-1,3-dimethyluracil and 1.33 g (6.45 mmol) of 4-methoxy-2,5-dimethylcinnamic acid. Then, the resultant crude crystals were recrystallized from dimethylformamide to give 393 mg (yield 20%) of Compound 29 as pale yellow grains.

Melting Point: >280°C

| Elemental Analysis: $C_{18}H_{20}N_4O_3$ | | | |
|---|---|---|---|
| Calcd. (%) | C, 63.51; | H, 5.92; | N, 16.46 |
| Found (%) | C, 63.59; | H, 6.10; | N, 16.23 |

IR (KBr) $\nu_{max}$ ($cm^{-1}$): 1703, 1648, 1509, 1260

NMR (270MHz; DMSO-$d_6$) $\delta$ (ppm) : 13.48(1H, brs), 7.81 (1H, d, J=16.2Hz), 7.50(1H, s), 6.82(1H, d, J=16.2Hz), 6.81(1H, s), 3.81(3H, s), 3.46(3H, s), 3.25(3H, s), 2.40(3H, s), 2.14(3H, s)

Example 30

(E)-8-(4-Methoxy-2,5-dimethylstyryl)caffeine (Compound 30)

Substantially the same procedure as in Example 2 was repeated using 300 mg (0.88 mmol) of Compound 29 obtained in Example 29 in place of Compound 1. Then, the resultant crude crystals were recrystallized from dioxane to give 211 mg (yield 68%) of Compound 30 as yellow needles.

Melting Point: >280°C

MS-EI m/e: 354($M^+$), 339($M^+$-$CH_3$)

IR (KBr) $\nu_{max}$ ($cm^{-1}$): 1692, 1653, 1508

NMR (270MHz; $CDCl_3$) $\delta$ (ppm): 8.00(1H, d, J=15.3Hz), 7.42(1H, s), 6.72(1H, d, J=15.3Hz), 6.66(1H, s), 4.06 (3H, s), 3.86(3H, s), 3.64(3H, s), 3.42(3H, s), 2.49(3H, s), 2.23(3H, s)

Example 31

(E)-8-(2,4-Dimethoxy-3-methylstyryl)theophylline (Compound 31)

Substantially the same procedure as in Example 1 was repeated using 1.0 g (5.88 mmol) of 5,6-diamino-1,3-dimethyluracil and 1.44 g (6.45 mmol) of 2,4-dimethoxy-3-methylcinnamic acid. Then, the resultant crude crystals were recrystallized from dioxane to give 581 mg (yield 28%) of Compound 31 as pale yellow needles.

Melting Point: >280°C

| Elemental Analysis: $C_{18}H_{20}N_4O_4$ | | | |
|---|---|---|---|
| Calcd. (%) | C, 60.67; | H, 5.65; | N, 15.72 |
| Found (%) | C, 60.34; | H, 5.77; | N, 15.64 |

IR (KBr) $\nu_{max}$ ($cm^{-1}$): 1695, 1653, 1499, 1270

NMR (270MHz; DMSO-$d_6$) $\delta$ (ppm): 13.52(1H, brs), 7.75 (1H, d, J=16.2Hz), 7.55(1H, d, J=8.3Hz), 6.96(1H, d, J=16.2Hz), 6.84(1H, d, J=8.3Hz), 3.83(3H, s), 3.70(3H, s), 3.47(3H, s), 3.25(3H, s), 2.09(3H, s)

Example 32

(E)-8-(2,4-Dimethoxy-3-methylstyryl)caffeine (Compound 32)

Substantially the same procedure as in Example 2 was repeated using 300 mg (0.84 mmol) of Compound 31 obtained in Example 31 in place of Compound 1. Then, the resultant crude crystals were recrystallized from methylene chloride/diethyl ether to give 239 mg (yield 77%) of Compound 32 as white needles.

Melting Point: 252.7-253.5°C

| Elemental Analysis: $C_{19}H_{22}N_4O_4$ | | | |
|---|---|---|---|
| Calcd. (%) | C, 61.61; | H, 5.98; | N, 15.13 |
| Found (%) | C, 61.40; | H, 6.06; | N, 15.17 |

IR (KBr) $\nu_{max}$ ($cm^{-1}$): 1692, 1651, 1505

NMR (270MHz; $CDCl_3$) $\delta$ (ppm): 7.92(1H, d, J=15.8Hz), 7.42(1H, d, J=8.9Hz), 6.99(1H, d, J=15.8Hz), 6.70 (IH, d, J=8.9Hz), 4.04(3H, s), 3.88(3H, s), 3.78 (3H, s), 3.64(3H, s), 3.42(3H, s), 2.19(3H, s)

Example 33

(E)-8-(2,5-Dimethylstyryl)-1,3-diethylxanthine (Compound 33)

Substantially the same procedure as in Example 1 was repeated using 3.00 g (15.1 mmol) of 5,6-diamino-1,3-diethyluracil and 3.20 g (18.2 mmol) of 2,5-dimethylcinnamic acid. Then, the resultant crude crystals were recrystallized from ethanol/toluene to give 2.56 g (yield 50%) of Compound 33 as white needles.

Melting Point: 281.8-282.5°C

| Elemental Analysis: $C_{19}H_{22}N_4O_2 \cdot 0.5H_2O$ | | | |
|---|---|---|---|
| Calcd. (%) | C, 66.46; | H, 6.97; | N, 15.50 |
| Found (%) | C, 66.77; | H, 6.82; | N, 15.72 |

IR (KBr) $\nu_{max}$ ($cm^{-1}$): 1706, 1639, 1503

NMR (270MHz; DMSO-$d_6$) $\delta$ (ppm): 7.84(1H, d, J=16.3Hz), 7.53(1H, s), 7.13(1H, d, J=7.4Hz), 7.06(1H, d, J=7.4Hz), 7.00(1H, d, J=16.3Hz), 4.06(2H, q, J=7.1Hz), 3.94(2H, q, J=7.lHz), 2.37(3H, s), 2.30 (3H, s), 1.26(3H, t, J=7.1Hz), 1.14(3H, t, J=7.1Hz)

Example 34

(E)-8-(2,5-Dimethylstyryl)-1,3-diethyl-7-methylxanthine (Compound 34)

Substantially the same procedure as in Example 2 was repeated using 2.00 g (5.92 mmol) of Compound 33 obtained in Example 33 in place of Compound 1. Then, the resultant crude crystals were recrystallized from toluene/cyclohexane to give 1.29 g (yield 62%) of Compound 34 as white needles.

Melting Point: 190.3-190.7°C

| Elemental Analysis: $C_{20}H_{24}N_4O_2$ | | | |
|---|---|---|---|
| Calcd. (%) | C, 68.16; | H, 6.86; | N, 15.89 |
| Found (%) | C, 68.15; | H, 7.02; | N, 15.65 |

IR (KBr) $\nu_{max}$ ($cm^{-1}$): 1698, 1657

NMR (270MHz; $CDCl_3$) $\delta$ (ppm): 7.86(1H, d, J=15.8Hz), 7.71(1H, s), 7.23(1H, d, J=15.8Hz), 7.15(1H, d, J=7.9Hz), 7.09(1H, d, J=7.9Hz), 4.11-4.04(2H, m), 4.04(3H, s), 3.92(2H, q, J=6.9Hz), 2.37(3H, s), 2.32(3H, s), 1.26(3H, t, J=6.9Hz), 1.13(3H, t, J=6.9Hz)

Example 35

(E)-8-(4-Ethoxystyryl)-1,3-diethylxanthine (Compound 35)

Substantially the same procedure as in Example 1 was repeated using 3.00 g (15.1 mmol) of 5,6-diamino-1,3-diethyluracil and 3.20 g (16.7 mmol) of 4-ethoxycinnamic acid. Then, the resultant crude crystals were recrystallized from dioxane to give 2.97 g (yield 55%) of Compound 35 as pale yellow needles.

Melting Point: 296.7-298.6°C

| Elemental Analysis: $C_{19}H_{22}N_4O_3$ | | | |
|---|---|---|---|
| Calcd. (%) | C, 64.39; | H, 6.25; | N, 15.81 |
| Found (%) | C, 64.54; | H, 6.52; | N, 15.80 |

IR (KBr) $\nu_{max}$ ($cm^{-1}$): 1695, 1647, 1516, 1250

NMR (270MHz; DMSO-$d_6$) δ (ppm): 13.36(1H, brs), 7.59 (1H, d, J=16.2Hz), 7.55(2H, d, J=8.6Hz), 6.96(2H, d, J=8.6Hz), 6.88(1H, d, J=16.2Hz), 4.11-4.04(4H, m), 3.94(2H, q, J=6.9Hz), 1.34(3H, t, J=6.9Hz), 1.26(3H, t, J=6.9Hz), 1.14(3H, t, J=6.9Hz)

Example 36

(E)-8-(4-Ethoxystyryl)-1,3-diethyl-7-methylxanthine (Compound 36)

Substantially the same procedure as in Example 2 was repeated using 1.60 g (4.52 mmol) of Compound 35 obtained in Example 35 in place of Compound 1. Then, the resultant crude crystals were recrystallized from ethyl acetate to give 1.47 g (yield 88%) of Compound 36 as pale green needles.

Melting Point: 185.3-185.7°C

| Elemental Analysis: $C_{20}H_{24}N_4O_3$ | | | |
|---|---|---|---|
| Calcd. (%) | C, 65.20; | H, 6.56; | N, 15.21 |
| Found (%) | C, 65.28; | H, 6.85; | N, 15.18 |

IR (KBr) $\nu_{max}$ ($cm^{-1}$): 1693, 1666, 1515, 1248

NMR (270MHz; $CDCl_3$) δ (ppm): 7.74(1H, d, J=15.8Hz), 7.52(2H, d, J=8.6Hz), 6.92(2H, d, J=8.6Hz), 6.77 (1H, d, J=15.8Hz), 4.21(2H, q, J=6.9Hz), 4.12-4.01 (4H, m), 4.04(3H, s), 1.44(3H, t, J=6.9Hz), 1.38 (3H, t, J=7.6Hz), 1.26 (3H, t, J=6.9Hz)

Example 37

(E)-1,3-Diethyl-8-(4-propoxystyryl)xanthine (Compound 37)

Substantially the same procedure as in Example 1 was repeated using 3.00 g (15.1 mmol) of 5,6-diamino-1,3-diethyluracil and 3.43 g (16.6 mmol) of 4-propoxycinnamic acid. Then, the resultant crude crystals were recrystallized from dioxane/water to give 3.02 g (yield 54%) of Compound 37 as pale yellow needles.

Melting Point: >270°C

| Elemental Analysis: $C_{20}H_{24}N_4O_3$ | | | |
|---|---|---|---|
| Calcd. (%) | C, 65.20; | H, 6.56; | N, 15.21 |
| Found (%) | C, 64.91; | H, 6.79; | N, 15.14 |

IR (KBr) $\nu_{max}$ ($cm^{-1}$): 1695, 1656, 1515, 1250

NMR (270MHz; DMSO-$d_6$) δ (ppm): 13.38(1H, brs), 7.59 (1H, d, J=16.5Hz), 7.55(2H, d, J=8.6Hz), 6.97(2H, d, J=8.6Hz), 6.87(1H, d, J=16.5Hz), 4.07(2H, q, J=7.3Hz), 4.00-3.90(4H, m), 1.81-1.67(2H, m), 1.26 (3H, t, J=6.9Hz), 1.14(3H, t, J=6.9Hz), 0.98(3H, t, J=7.3Hz)

Example 38

(E)-1,3-Diethyl-7-methyl-8-(4-propoxystyryl)xanthine (Compound 38)

Substantially the same procedure as in Example 2 was repeated using 1.70 g (4.61 mmol) of Compound 37 obtained in Example 37 in place of Compound 1. Then, the resultant crude crystals were recrystallized from hexane/ethyl acetate to give 1.37 g (yield 78%) of Compound 38 as pale yellow needles.

Melting Point: 155.7-156.5°C

| Elemental Analysis: $C_{21}H_{26}N_4O_3$ | | | |
|---|---|---|---|
| Calcd. (%) | C, 65.92; | H, 6.85; | N, 14.65 |
| Found (%) | C, 65.72; | H, 7.05; | N, 14.59 |

IR (KBr) $\nu_{max}$ ($cm^{-1}$): 1696, 1665, 1513, 1246

NMR (270MHz; $CDCl_3$) δ (ppm): 7.74(1H, d, J=15.8Hz), 7.52(2H, d, J=8.6Hz), 6.92(2H, d, J=8.6Hz), 6.77 (1H, d, J=15.8Hz), 4.21(2H, q, J=6.9Hz), 4.09(2H, q, J=6.9Hz), 4.04(3H, s), 3.97(2H, t, J=6.6Hz), 1.90-1.77(2H, m), 1.38 (3H, t, J=6.9Hz), 1.26(3H, t, J=6.9Hz), 1.05(3H, t, J=7.3Hz)

Example 39

(E)-1,3-Diethyl-8-(3-methoxystyryl)xanthine (Compound 39)

Substantially the same procedure as in Example 1 was repeated using 2.50 g (12.6 mmol) of 5,6-diamino-1,3-diethyluracil and 2.48 g (13.9 mmol) of 3-methoxycinnamic acid. Then, the resultant crude crystals were recrystallized from dimethylformamide/water to give 2.10 g (yield 49%) of Compound 39 as a white powder.

Melting Point: 270.6-272.5°C

| Elemental Analysis: $C_{18}H_{20}N_4O_3$ | | | |
|---|---|---|---|
| Calcd. (%) | C, 63.52; | H, 5.92; | N, 16.46 |
| Found (%) | C, 63.20; | H, 6.01; | N, 16.34 |

IR (KBr) $\nu_{max}$ ($cm^{-1}$): 1686, 1634, 1500

NMR (270MHz; DMSO-$d_6$) δ (ppm): 7.61(1H, d, J=16.4Hz), 7.34(1H, t, J=7.9Hz), 7.20-7.18(2H, m), 7.07(1H, d, J=16.4Hz), 6.92(1H, d, J=8.6Hz), 4.06(2H, q, J=7.0Hz), 3.94(2H, q, J=6.8Hz), 1.26(3H, t, J=7.0Hz), 1.14(3H, t, J=6.8Hz)

Example 40

(E)-1,3-Diethyl-8-(3-methoxystyryl)-7-methylxanthine (Compound 40)

Substantially the same procedure as in Example 2 was repeated using 1.70 g (5.00 mmol) of Compound 39 obtained in Example 39 in place of Compound 1. Then, the resultant crude crystals were recrystallized from toluene/cyclohexane to give 1.10 g (yield 62%) of Compound 40 as pale yellow needles.

Melting Point: 153.4-154.8°C

| Elemental Analysis: $C_{19}H_{22}N_4O_3$ | | | |
|---|---|---|---|
| Calcd. (%) | C, 64.39; | H, 6.26; | N, 15.81 |
| Found (%) | C, 64.34; | H, 6.38; | N, 15.82 |

IR (KBr) $\nu_{max}$ ($cm^{-1}$): 1692, 1656, 1541

NMR (270MHz; DMSO-$d_6$) δ (ppm): 7.64(1H, d, J=15.8Hz), 7.40-7.30(4H, m), 6.97-6.92(1H, m), 4.31-4.05(2H, m), 4.05(3H, s), 3.92(2H, q, J=7.0Hz), 1.26(3H, t, J=7.1Hz), 1.13(3H, t, J=7.0Hz)

Example 41

(E)-8-(4-Butoxystyryl)-1,3-diethylxanthine (Compound 41)

Substantially the same procedure as in Example 1 was repeated using 3.00 g (15.1 mmol) of 5,6-diamino-1,3-diethyluracil and 3.67 g (16.7 mmol) of 4-butoxycinnamic acid. Then, the resultant crude crystals were recrystallized from dioxane/water to give 3.04 g (yield 53%) of Compound 41 as pale yellow needles.

Melting Point: 257.9-261.3°C

| Elemental Analysis: $C_{21}H_{26}N_4O_3$ | | | |
|---|---|---|---|
| Calcd. (%) | C, 65.95; | H, 6.85; | N, 14.65 |
| Found (%) | C, 65.90; | H, 7.21; | N, 14.60 |

IR (KBr) $\nu_{max}$ ($cm^{-1}$): 1695, 1645, 1515, 1248

NMR (270MHz; DMSO-$d_6$) δ (ppm): 13.32(1H, brs), 7.59 (1H, d, J=16.5Hz), 7.55(2H, d, J=8.9Hz), 6.97(2H, d, J=8.9Hz), 6.87(1H, d, J=16.5Hz), 4.10-3.90(6H, m), 1.76-1.66(2H, m), 1.51-1.40(2H, m), 1.26(3H, t, J=6.9Hz), 1.14 (3H, t, J=6.9Hz), 0.94(3H, t, J=7.3Hz)

Example 42

(E)-8-(4-Butoxystyryl)-1,3-diethyl-7-methylxanthine (Compound 42)

Substantially the same procedure as in Example 2 was repeated using 1.50 g (3.92 mmol) of Compound 41 obtained in Example 41 in place of Compound 1. Then, the resultant crude crystals were recrystallized from hexane/ethyl acetate to give 982 mg (yield 63%) of Compound 42 as pale yellow needles.

Melting Point: 123.4-123.6°C

| Elemental Analysis: $C_{22}H_{28}N_4O_3$ | | | |
|---|---|---|---|
| Calcd. (%) | C, 66.65; | H, 7.11; | N, 14.13 |
| Found (%) | C, 66.81; | H, 7.31; | N, 14.01 |

IR (KBr) $\nu_{max}$ ($cm^{-1}$): 1693, 1665, 1513, 1251

NMR (270MHz; $CDCl_3$) δ (ppm): 7.74(1H, d, J=15.8Hz), 7.52(2H, d, J=8.9Hz), 6.92(2H, d, J=8.9Hz), 6.76 (1H, d, J=15.8Hz), 4.21(2H, q, J=6.9Hz), 4.09(2H, q, J=6.9Hz), 4.04(3H, s), 4.02(2H, q, J=6.6Hz), 1.84-1.74(2H, m), 1.58-1.44(2H, m), 1.38(3H, t, J=6.9Hz), 1.26(3H, t, J=6.9Hz), 0.99(3H, t, J=7.3Hz)

Example 43

(E)-1,3-Diethyl-8-(4-methylstyryl)xanthine (Compound 43)

Substantially the same procedure as in Example 1 was repeated using 3.00 g (15.1 mmol) of 5,6-diamino-1,3-diethyluracil and 2.70 g (16.7 mmol) of 4-methylcinnamic acid. Then, the resultant crude crystals were recrystallized from dioxane to give 2.64 g (yield 54%) of Compound 43 as pale yellow needles.

Melting Point: >280°C

| Elemental Analysis: $C_{18}H_{20}N_4O_2$ | | | |
|---|---|---|---|
| Calcd. (%) | C, 66.65; | H, 6.21; | N, 17.27 |
| Found (%) | C, 66.53; | H, 6.27; | N, 17.14 |

IR (KBr) $\nu_{max}$ ($cm^{-1}$): 1692, 1644, 1518, 1490

NMR (270MHz; DMSO-$d_6$) δ (ppm): 13.53(1H, brs), 7.62 (1H, d, J=16.5Hz), 7.52(2H, d, J=7.9Hz), 7.24(2H, d, J=7.9Hz), 6.98(1H, d, J=16.5Hz), 4.07(2H, q, J=6.9Hz), 3.94(2H, q, J=6.9Hz), 2.33(3H, s), 1.26 (3H, t, J=6.9Hz), 1.14 (3H, t, J=6.9Hz)

Example 44

(E)-1,3-Diethyl-7-methyl-8-(4-methylstyryl)xanthine (Compound 44)

Substantially the same procedure as in Example 2 was repeated using 1.50 g (4.62 mmol) of Compound 43 obtained in Example 43 in place of Compound 1. Then, the resultant crude crystals were recrystallized from dioxane/water to give 1.39 g (yield 89%) of Compound 44 as yellow needles.

Melting Point: 170.8-171.5°C

| Elemental Analysis: $C_{19}H_{22}N_4O_2$ | | | |
|---|---|---|---|
| Calcd. (%) | C, 67.44; | H, 6.55; | N, 16.56 |
| Found (%) | C, 67.58; | H, 6.65; | N, 16.68 |

IR (KBr) $\nu_{max}$ ($cm^{-1}$): 1687, 1650, 1542, 1516

NMR (270MHz; $CDCl_3$) δ (ppm): 7.77(1H, d, J=15.8Hz), 7.48(2H, d, J=8.3Hz), 7.21(2H, d, J=8.3Hz), 6.87 (1H, d, J=15.8Hz), 4.22(2H, q, J=6.9Hz), 4.09(2H, q, J=6.9Hz), 4.05(3H, s), 2.39(3H, s), 1.38(3H, t, J=6.9Hz), 1.26(3H, t, J=6.9Hz)

Example 45

(E)-1,3-Diethyl-8-(2-methoxystyryl)xanthine (Compound 45)

Substantially the same procedure as in Example 1 was repeated using 2.5 g (12.6 mmol) of 5,6-diamino-1,3-diethyluracil and 2.48 g (13.9 mmol) of 2-methoxycinnamic acid. Then, the resultant crude crystals were recrystallized from tetrahydrofuran/water to give 990 mg (yield 24%) of Compound 45 as yellow grains.

Melting Point: >270°C

| Elemental Analysis: $C_{18}H_{20}N_4O_3$ | | | |
|---|---|---|---|
| Calcd. (%) | C, 63.52; | H, 5.92; | N, 16.46 |
| Found (%) | C, 63.28; | H, 5.86; | N, 16.43 |

IR (KBr) $\nu_{max}$ ($cm^{-1}$): 1694, 1640, 1501

NMR (270MHz; DMSO-$d_6$) δ (ppm): 7.85(1H, d, J=16.8Hz), 7.62(1H, d, J=7.6Hz), 7.34(1H, t, J=7.6Hz), 7.11-6.98 (3H, m), 4.07(2H, q, J=7.0Hz), 3.97-3.89(2H, m), 3.89(3H, s), 1.26(3H, t, J=7.0Hz), 1.14(3H, t, J=6.9Hz)

Example 46

(E)-1,3-Diethyl-8-(2-methoxystyryl)-7-methylxanthine (Compound 46)

Substantially the same procedure as in Example 2 was repeated using 1.5 g (4.41 mmol) of Compound 45 obtained in Example 45 in place of Compound 1. Then, the resultant crude crystals were recrystallized from ethanol/water to give 800 mg (yield 51%) of Compound 46 as yellow needles.

Melting Point: 189.6-190.0°C

| Elemental Analysis: $C_{19}H_{22}N_4O_3$ | | | |
|---|---|---|---|
| Calcd. (%) | C, 64.39; | H, 6.26; | N, 15.81 |
| Found (%) | C, 64.18; | H, 6.25; | N, 15.77 |

IR (KBr) $\nu_{max}$ ($cm^{-1}$): 1697, 1649

NMR (270MHz; DMSO-$d_6$) δ (ppm): 7.94(1H, d, J=15.8Hz), 7.88(1H, dd, J=7.9, 1.5Hz), 7.41-7.34(1H, m), 7.31 (1H, d, J=15.8Hz), 7.10(1H, d, J=7.9Hz), 7.02(1H, t, J=7.4Hz), 4.11-4.02(2H, m), 4.02(3H, s), 3.96-3.90(2H, m), 3.90 (3H, s), 1.29(3H, t, J=7.2Hz), 1.13(3H, t, J=7.2Hz)

Example 47

(E)-1,3-Diethyl-8-(4-methoxy-3-methylstyryl)xanthine (Compound 47)

Substantially the same procedure as in Example 1 was repeated using 2.50 g (12.6 mmol) of 5,6-diamino-1,3-diethyluracil and 3.00 g (13.9 mmol) of 4-methoxy-3-methylcinnamic acid. Then, the resultant crude crystals were recrystallized from dimethylsulfoxide/water to give 1.70 g (yield 36%) of Compound 47 as white flocculent precipitates.

Melting Point: >270°C

| Elemental Analysis: $C_{19}H_{22}N_4O_3$ | | | |
|---|---|---|---|
| Calcd. (%) | C, 64.39; | H, 6.23; | N, 15.81 |
| Found (%) | C, 64.05; | H, 6.34; | N, 15.74 |

IR (KBr) $\nu_{max}$ ($cm^{-1}$): 1689, 1644, 1510, 1459

NMR (270MHz; DMSO-$d_6$) δ (ppm): 7.56(1H, d, J=16.3Hz), 7.45(1H, s), 7.44(1H, d, J=8.2Hz), 6.98(1H, d, J=8.2Hz), 6.87(1H, d, J=16.3Hz), 4.06(2H, q, J=7.1Hz), 3.93(2H, q, J=7.0Hz), 3.82(3H, s), 2.18 (3H, s), 1.25(3H, t, J=7.1Hz), 1.13(3H, t, J=7.0Hz)

Example 48

(E)-1,3-Diethyl-8-(4-methoxy-3-methylstyryl)-7-methylxanthine (Compound 48)

Substantially the same procedure as in Example 2 was repeated using 1.27 g (3.36 mmol) of Compound 47 obtained in Example 47 in place of Compound 1. Then, the resultant crude crystals were recrystallized from toluene/cyclohexane to give 1.01 g (yield 82%) of Compound 48 as yellow needles.

Melting Point: 176.5-177.6°C

| Elemental Analysis: $C_{20}H_{24}N_4O_3$ | | | |
|---|---|---|---|
| Calcd. (%) | C, 65.20; | H, 6.57; | N, 15.21 |
| Found (%) | C, 65.22; | H, 6.75; | N, 15.22 |

IR (KBr) $\nu_{max}$ ($cm^{-1}$): 1687, 1648, 1542, 1505, 1434

NMR (270MHz; DMSO-$d_6$) δ (ppm): 7.65(1H, s), 7.58(1H, d, J=15.8Hz), 7.57-7.53(1H, m), 7.16(1H, d, J=15.8Hz), 6.97(1H, d, J=8.9Hz), 4.10-4.01(2H, m), 4.01(3H, s), 3.91(2H, q, J=6.9Hz), 3.88(3H, s), 2.19(3H, s), 1.25(3H, t, J=6.9Hz), 1.12(3H, t, J=6.9Hz)

Example 53 Tablets

Tablets having the following composition were prepared in a conventional manner.

| Composition of One Tablet | |
|---|---|
| Compound 2 | 20 mg |
| Lactose | 143.4mg |
| Potato Starch | 30 mg |
| Hydroxypropylcellulose | 6 mg |
| Magnesium Stearate | 0.6mg |
| | 200 mg |

Example 55 Capsules

Capsules having the following composition were prepared in a conventional manner.

| Composition of One Capsule | |
|---|---|
| Compound 8 | 20 mg |
| Avicel | 99.5mg |
| Magnesium Stearate | 0.5mg |
| | 120 mg |

Example 56 Injections

Injections having the following composition were prepared in a conventional manner.

| Composition of One Injection Vial | |
|---|---|
| Compound 10 | 2 mg |
| Purified Soybean Oil | 200 mg |
| Purified Egg Yolk Lecithin | 24 mg |
| Glycerine for Injection | 50 mg |
| Distilled Water for Injection | 1.72 ml |
| | 2.00 ml |

Example 57 Syrup Preparations

Syrup Preparations having the following composition were prepared in a conventional manner.

| Composition of One Syrup Preparation | |
|---|---|
| Compound 14 | 20 mg |
| Refined Sugar | 30 mg |
| Ethyl p-Hydroxybenzoate | 40 mg |
| Propyl p-Hydroxybenzoate | 10 mg |
| Strawberry Flavor | 0.1 ml |
| Water | 99.8 ml |
| | 100 ml |

Reference Example 19

(E)-8-(3,5-Dimethoxystyryl)-1,3-diethylxanthine (Compound 67)

Substantially the same procedure as in Example 1 was repeated using 3.00 g (15.1 mmol) of 5,6-diamino-1,3-diethyluracil and 3.48 g (16.7 mmol) of 3,5-dimethoxycinnamic acid. Then, the resultant crude crystals were recrystallized from ethanol/water to give 2.74 g (yield 49%) of Compound 67 as a white powder.

Melting Point: >270°C

| Elemental Analysis: $C_{19}H_{22}N_4O_4 \cdot 0.5H_2O$ | | | |
|---|---|---|---|
| Calcd. (%) | C, 60.15; | H, 6.11; | N, 14.77 |
| Found (%) | C, 60.41; | H, 6.15; | N, 15.02 |

IR (KBr) $\nu_{max}$ ($cm^{-1}$): 1686, 1638, 1587

NMR (270MHz; DMSO-$d_6$) δ (ppm): 7.57(1H, d, J=16.5Hz), 7.07(1H, d, J=16.5Hz), 6.79 (2H, d, J=2.0Hz), 6.50 (1H, t, J=2.0Hz), 4.06(2H, q, J=7.0Hz), 3.94(2H, q, J=6.9Hz), 3.79(6H,s), 1.26(3H, t, J=7.0Hz), 1.14(3H, t, J=6.9Hz)

Reference Example 20

(E)-8-(3,5-Dimethoxystyryl)-1,3-diethyl-7-methylxanthine (Compound 68)

Substantially the same procedure as in Example 2 was repeated using 3.00 g (8.11 mmol) of Compound 67 obtained in Reference Example 19 in place of Compound 1. Then, the resultant crude crystals were recrystallized from toluene/cyclohexane to give 2.28 g (yield 73%) of Compound 68 as yellow needles.

Melting Point: 184.2-185.3°C

| Elemental Analysis: $C_{20}H_{24}N_4O_4$ | | | |
|---|---|---|---|
| Calcd. (%) | C, 62.49; | H, 6.29; | N, 14.57 |
| Found (%) | C, 62.66; | H, 6.48; | N, 14.65 |

IR (KBr) $\nu_{max}$ ($cm^{-1}$): 1690, 1659, 1595

NMR (270MHz; DMSO-$d_6$) δ (ppm): 7.60(1H, d, J=15.7Hz), 7.35(1H, d, J=15.7Hz), 6.98(2H, d, J=2.2Hz), 6.51 (1H, t, J=2.2Hz), 4.11-4.01(2H, m), 4.05(3H, s), 3.92(2H, q, J=7.0Hz), 3.80(6H, s), 1.26(3H, t, J=7.1Hz), 1.13(3H, t, J=7.0Hz)

Reference Example 33

(E)-1,3-Diethyl-8-(4-methoxymethoxystyryl)xanthine (Compound 81)

Substantially the same procedure as in Example 1 was repeated using 4.00 g (20.2 mmol) of 5,6-diamino-1,3-diethyluracil and 4.62 g (22.2 mmol) of 4-methoxymethoxycinnamic acid. Then, the resultant crude crystals were recrystallized from dioxane/water to give 4.80 g (yield 64%) of Compound 81 as pale yellow needles.

Melting Point: 270.2-271.4°C

| Elemental Analysis: $C_{19}H_{22}N_4O_4$ | | | |
|---|---|---|---|
| Calcd. (%) | C, 61.61; | H, 5.98; | N, 15.13 |
| Found (%) | C, 61.97; | H, 5.98; | N, 15.05 |

IR (KBr) $\nu_{max}$ ($cm^{-1}$): 1695, 1641, 1510, 1238

NMR (270MHz; DMSO-$d_6$) δ (ppm) : 13.40(1H, brs), 7.60 (1H, d, J=16.5Hz), 7.57(2H, d, J=8.6Hz), 7.06(2H, d, J=8.6Hz), 6.90(1H, d, J=16.5Hz), 5.23(2H, s), 4.07(2H, q, J=6.9Hz), 3.94(2H, q, J=6.9Hz), 3.39 (3H, s), 1.26(3H, t, J=6.9Hz), 1.14(3H, t, J=6.9Hz)

Reference Example 34

(E)-1,3-Diethyl-8-(4-methoxymethoxystyryl)-7-methylxanthine (Compound 82)

Substantially the same procedure as in Example 2 was repeated using 3.50 g (9.45 mmol) of Compound 81 obtained in Reference Example 33 in place of Compound 1. Then, the resultant crude crystals were recrystallized from hexane/ethyl acetate to give 3.39 g (yield 93%) of Compound 82 as pale yellow plates.

Melting Point: 163.9-164.7°C

| Elemental Analysis: $C_{20}H_{24}N_4O_4$ | | | |
|---|---|---|---|
| Calcd. (%) | C, 62.49; | H, 6.29; | N, 14.57 |
| Found (%) | C, 62.21; | H, 6.27; | N, 14.58 |

IR (KBr) $\nu_{max}$ ($cm^{-1}$): 1688, 1651, 1510, 1238

NMR (270MHz; $CDCl_3$) δ (ppm): 7.75(1H, d, J=15.8Hz), 7.53(2H, d, J=8.6Hz), 7.07(2H, d, J=8.6Hz), 6.79 (1H, d, J=15.8Hz), 5.21(2H, s), 4.21(2H, q, J=6.9Hz), 4.09(2H, q, J=6.9Hz), 4.05(3H, s), 3.50 (3H, s), 1.38(3H, t, J=6.9Hz), 1.26(3H, t, J=6.9Hz)

**Claims**

1. Use of a xanthine derivative of Formula (I):

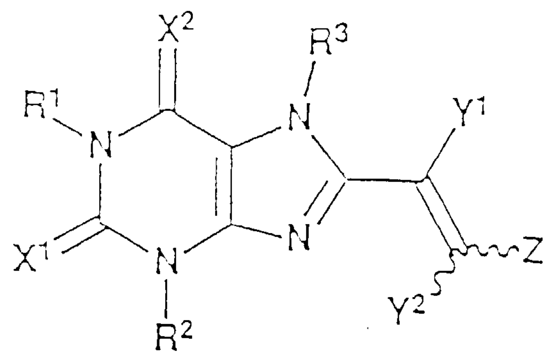

I

in which:

$R^1$ and $R^2$ are independently methyl or ethyl;
$R^3$ is hydrogen, $C_1$-$C_6$ straight or branched chain alkyl
$Y^1$ and $Y^2$ are hydrogen, and Z represents a

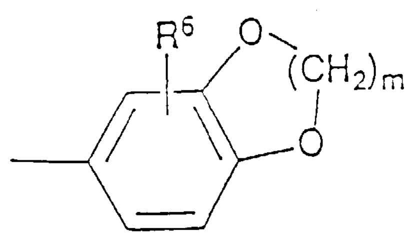

group in which $R^6$ is hydrogen, and m represents 1 or 2;
and $X^1$ and $X^2$ are O;

or a pharmaceutically acceptable salt thereof, for the manufacture of a medicament for the treatment of Parkinson's disease.

2. Use according to claim 1 wherein $R^3$ is a $C_1$-$C_6$ alkyl group.

3. Use according to claim 2 wherein $R^3$ is methyl.

4. Use according to claim 1, 2 or 3 wherein the configuration at the position 8 of the xanthine ring is (E) form.

5. Use according to any one of claims 1 to 4 wherein $R^3$ is methyl; Z is

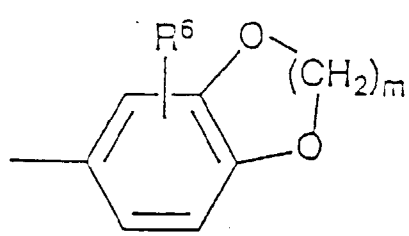

where $R^6$ is hydrogen; and m is 1 or 2.

6. A xanthine derivative of the formula (I-A):

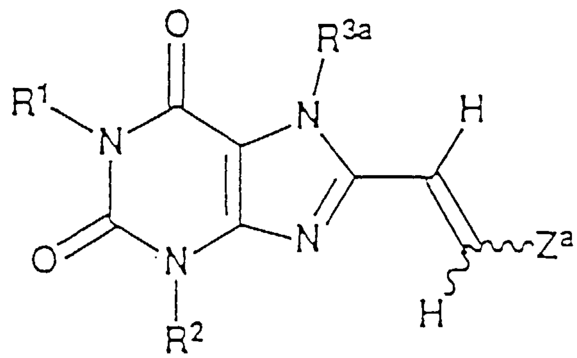

(I-A)

in which:

$R^1$ and $R^2$ are as defined in claim 1;
$R^{3a}$ is hydrogen or $C_1$-$C_6$ straight or branched chain alkyl; and
$Z^a$ is a

130

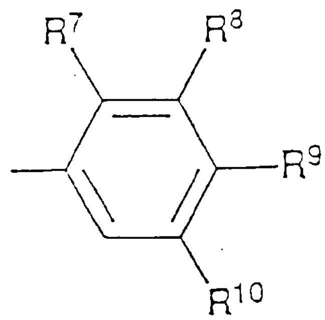

group wherein at least one of $R^7$, $R^8$ and $R^9$ groups is a $C_1$-$C_6$ straight or branched chain alkyl or alkoxy group and the others are hydrogen; and $R^{10}$ is hydrogen or $C_1$-$C_6$ straight or branched chain alkyl; or a

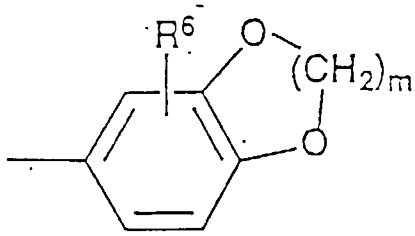

group wherein $R^6$ and m are as defined in claim 1;

or a pharmaceutically acceptable salt thereof.

**7.** A xanthine derivative according to claim 6, in which the configuration at the position 8 of the xanthine ring is (E) form.

**8.** A xanthine derivative according to claim 7, in which $Z^a$ is a

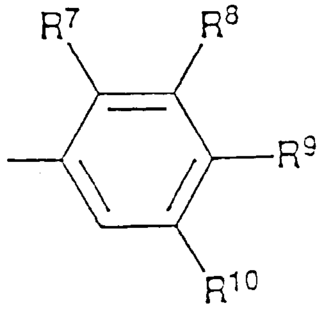

group where $R^7$, $R^8$, $R^9$ and $R^{10}$ are as defined in claim 6.

**9.** A xanthine derivative according to claim 6, 7, 8 wherein $R^{3a}$ is $C_1$-$C_6$ straight or branched chain alkyl.

**10.** A xanthine derivative according to claim 9 wherein $R^{3a}$ is methyl.

**11.** A xanthine derivative according to claim 6, 7, 8, 9 or 10 wherein one of $R^7$, $R^8$ and $R^9$ is methyl, methoxy, ethoxy or propoxy and the others are hydrogen; and $R^{10}$ is hydrogen or methyl.

**12.** A xanthine derivative according to any one of claims 6 to 11 wherein $R^1$ and $R^2$ are ethyl; $R^8$ and $R^9$ are methoxy; and $R^7$ and $R^{10}$ are hydrogen.

**13.** A xanthine derivative according to claim 6 or 7 wherein $R^{3a}$ is methyl; $Z^a$ is a

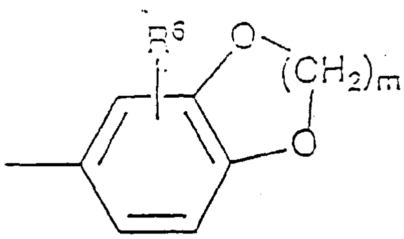

group wherein $R^6$ is hydrogen; and m is 1 or 2.

**14.** A pharmaceutical composition containing a compound or salt as claimed in any one of claims 6 to 13 in admixture with a pharmaceutically acceptable carrier or diluent.

### Patentansprüche

**1.** Verwendung eines Xanthinderivates der Formel (I):

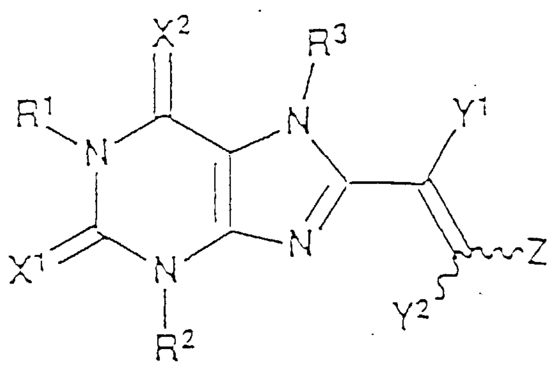

I

in welchem:

$R^1$ und $R^2$ unabhängig voneinander Methyl- oder Ethylgruppen sind;
$R^3$ ein Wasserstoffatom, ein unverzweigter oder verzweigter $C_1$-$C_6$-Alkylrest ist,
$Y^1$ und $Y^2$ Wasserstoffatome sind, und Z einen Rest

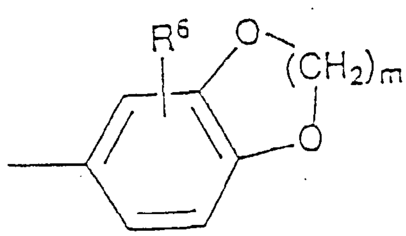

darstellt, in welchem $R^6$ ein Wasserstoffatom ist und m den Wert 1 oder 2 hat; und $X^1$ und $X^2$ Sauerstoffatome sind;

oder eines pharmazeutisch verträglichen Salzes davon, zur Herstellung eines Medikaments zur Behandlung von Morbus Parkinson.

2. Verwendung nach Anspruch 1, wobei $R^3$ ein $C_1$-$C_6$-Alkylrest ist.

3. Verwendung nach Anspruch 2, wobei $R^3$ eine Methylgruppe ist.

4. Verwendung nach Anspruch 1, 2 oder 3, wobei die Konfiguration an der Position 8 des Xanthinrings die (E) Form ist.

5. Verwendung nach einem der Ansprüche 1 bis 4, wobei $R^3$ eine Methylgruppe ist, Z der Rest

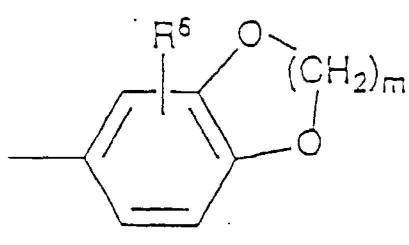

ist; $R^6$ ein Wasserstoffatom ist; und m 1 oder 2 ist.

6. Xanthinderivat der Formel (I-A):

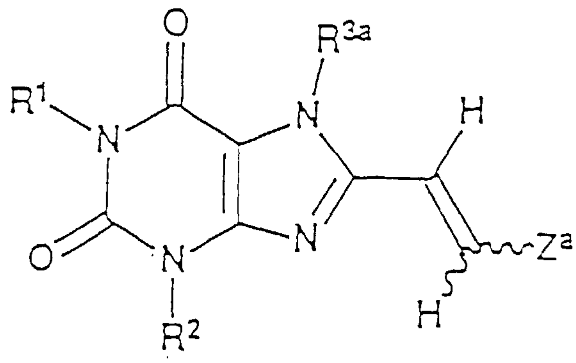

(I-A)

in welchem:

$R^1$ und $R^2$ wie in Anspruch 1 definiert sind;
$R^{3a}$ ein Wasserstoffatom oder ein unverzweigter oder verzweigter $C_1$-$C_6$-Alkylrest ist; und der Rest $Z^a$ ein Rest der Formel

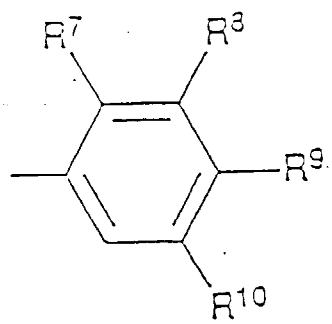

ist, in dem mindestens einer der Reste $R^7$, $R^8$ und $R^9$ ein unverzweigter oder verzweigter $C_1$-$C_6$-Alkyl- oder-Alkoxyrest ist und die anderen Reste Wasserstoffatome sind; und $R^{10}$ ein Wasserstoffatom oder ein unverzweigter oder verzweigter Alkylrest ist; oder ein Rest der Formel

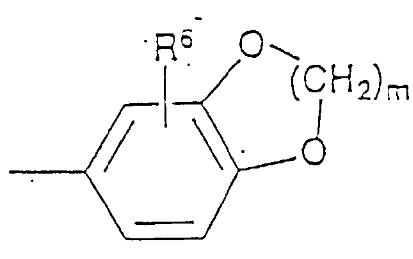

in dem $R^6$ und m wie in Anspruch 1 definiert sind;

oder ein pharmazeutisch verträgliches Salz davon.

**7.** Xanthinderivat nach Anspruch 6, in welchem die Konfiguration an der Position 8 des Xanthinrings die (E) Form ist.

**8.** Xanthinderivat nach Anspruch 7, in welchem $Z^a$ ein Rest der Formel

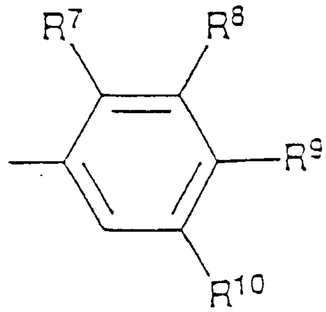

ist, in welchem $R^7$, $R^8$, $R^9$ und $R^{10}$ wie in Anspruch 6 definiert sind.

**9.** Xanthinderivat nach Anspruch 6, 7, 8, in welchem $R^{3a}$ ein unverzweigter oder verzweigter $C_1$-$C_6$- Alkylrest ist.

**10.** Xanthinderivat nach Anspruch 9, in dem $R^{3a}$ eine Methylgruppe ist.

**11.** Xanthinderivat nach Anspruch 6, 7, 8, 9 oder 10 wobei einer der Reste $R^7$, $R^8$ und $R^9$ eine Methyl-, Methoxy-, Ethoxy- oder Propoxygruppe ist und die anderen Reste Wasserstoffatome sind; und $R^{10}$ ein Wasserstoffatom oder eine Methylgruppe ist.

**12.** Xanthinderivat nach einem der Ansprüche 6 bis 11, in dem $R^1$ und $R^2$ Ethylgruppen sind; $R^8$ und $R^9$ Methoxygruppen sind; und $R^7$ und $R^{10}$ Wasserstoffatome sind.

**13.** Xanthinderivat nach Anspruch 6 oder 7, wobei $R^{3a}$ eine Methylgruppe ist, $Z^a$ ein Rest der Formel

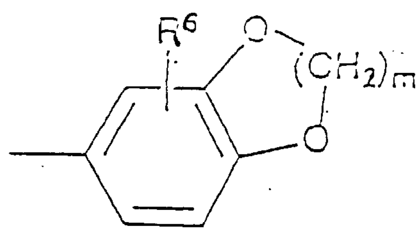

ist; $R^6$ ein Wasserstoffatom ist; und m 1 oder 2 ist.

**14.** Arzneimittel, enthaltend eine Verbindung oder Salz nach einem der Ansprüche 6 bis 13, zusammen mit einem pharmazeutisch verträglichen Träger oder Verdünnungsmittel.

**Revendications**

**1.** Emploi d'un dérivé de xanthine de formule (I) :

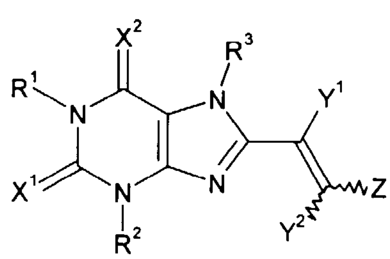

(I)

dans laquelle :

$R^1$ et $R^2$ représentent chacun, indépendamment, un groupe méthyle ou éthyle ;

$R^3$ représente un atome d'hydrogène ou un groupe alkyle en $C_{1-6}$ à chaîne droite ou ramifiée ;
$Y^1$ et $Y^2$ représentent des atomes d'hydrogène et Z représente un groupe

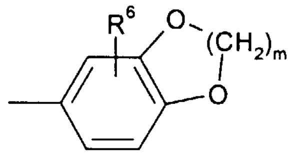

dans lequel $R^6$ représente un atome d'hydrogène et m représente 1 ou 2 ; et $X^1$ et $X^2$ représentent des atomes d'oxygène ;

ou d'un sel d'un tel composé, admissible en pharmacie, pour la fabrication d'un médicament destiné au traitement de la maladie de Parkinson.

**2.** Emploi conforme à la revendication 1, où $R^3$ représente un groupe alkyle en $C_{1-6}$.

**3.** Emploi conforme à la revendication 2, où $R^3$ représente un groupe méthyle.

**4.** Emploi conforme à la revendication 1, 2 ou 3, où la configuration adoptée par le cycle de xanthine en position (8) correspond à une forme (E).

**5.** Emploi conforme à l'une des revendications 1 à 4, où $R^3$ représente un groupe méthyle et Z représente un groupe de formule

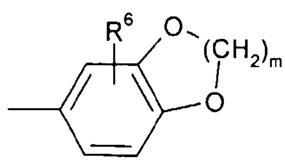

dans laquelle $R^6$ représente un atome d'hydrogène et m vaut 1 ou 2.

**6.** Dérivé de xanthine de formule (I-A):

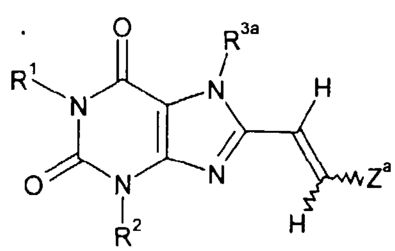

(I-A)

dans laquelle :

$R^1$ et $R^2$ ont les significations indiquées dans la revendication 1 ;
$R^{3a}$ représente un atome d'hydrogène ou un groupe alkyle en $C_{1-6}$ à chaîne droite ou ramifiée ; et
$Z^a$ représente un groupe de formule :

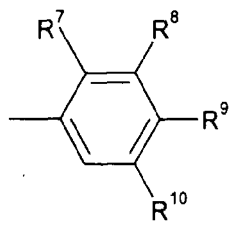

où au moins l'un des symboles $R^7$, $R^8$ et $R^9$ représente un groupe alkyle ou alcoxy en $C_{1-6}$ à chaîne droite ou ramifiée, les autres symboles représentant des atomes d'hydrogène, et $R^{10}$ représente un atome d'hydrogène ou un groupe alkyle en $C_{1-6}$ à chaîne droite ou ramifiée ; ou bien un groupe de formule :

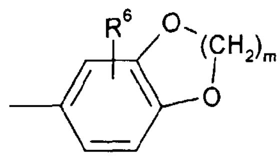

où $R^6$ et m ont les significations indiquées dans la revendication 1 ;

ainsi que des sels de tels composés, admissibles en pharmacie.

**7.** Dérivé de xanthine conforme à la revendication 6, dans lequel la configuration adoptée par le cycle de xanthine en position 8 correspond à une forme (E).

**8.** Dérivé de xanthine conforme à la revendication 7, dans lequel $Z^a$ représente un groupe de formule :

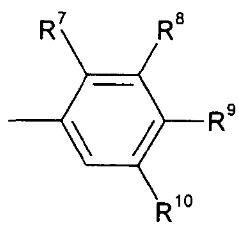

dans laquelle les symboles $R^7$, $R^8$, $R^9$ et $R^{10}$ ont les significations indiquées dans la revendication 6.

**9.** Dérivé de xanthine conforme à la revendication 6, 7 ou 8, dans lequel $R^{3a}$ représente un groupe alkyle en $C_{1-6}$ à chaîne droite ou ramifiée.

**10.** Dérivé de xanthine conforme à la revendication 9, dans lequel $R^{3a}$ représente un groupe méthyle.

**11.** Dérivé de xanthine conforme à la revendication 6, 7, 8, 9 ou 10, dans lequel l'un des symboles $R^7$, $R^8$ et $R^9$ représente un groupe méthyle, méthoxy, éthoxy ou propoxy, les autres représentant des atomes d'hydrogène, et $R^{10}$ représente un atome d'hydrogène ou un groupe méthyle.

**12.** Dérivé de xanthine conforme à l'une des revendications 6 à 11, dans lequel $R^1$ et $R^2$ représentent des groupes éthyle, $R^8$ et $R^9$ représentent des groupes méthoxy, et $R^7$ et $R^{10}$ représentent des atomes d'hydrogène.

**13.** Dérivé de xanthine conforme à la revendication 6 ou 7, dans lequel $R^{3a}$ représente un groupe méthyle et $Z^a$ représente un groupe de formule :

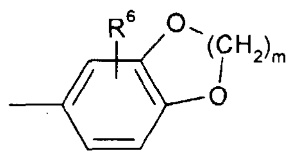

dans laquelle $R^6$ représente un atome d'hydrogène et m vaut 1 ou 2.

**14.** Composition pharmaceutique contenant un composé ou un sel conforme à l'une des revendications 6 à 13, mélangé avec un véhicule ou diluant admissible en pharmacie.